# EUROPEAN PATENT SPECIFICATION

(11) **EP 3 272 364 B1**
(45) Date of publication and mention of the grant of the patent: **29.07.2020**
(21) Application number: 17190590.4
(22) Date of filing: 12.12.2012
(51) Int. Cl.: A61P 35/00, A61P 35/02, A61P 43/00, A61K 39/00, A61K 51/10, C07K 16/28

(54) **CHIMERIC THERAPEUTIC ANTI-CD37 ANTIBODIE HH1**
CHIMÄRE THERAPEUTISCHE ANTIKÖRPER
ANTICORPS THÉRAPEUTIQUES CHIMÉRIQUES

(30) Priority: 13.12.2011 US 201161569981 P
(43) Date of publication of application: 24.01.2018
(62) Divisional of application: 12818820.8
(73) Proprietor: Nordic Nanovector ASA, 0884 Oslo (NO)
(72) Inventor: LARSEN, Roy Hartvig, 0598 Oslo (NO); DAHLE, Jostein, 1344 Haslum (NO)
(74) Representative: Aera A/S

(56) References cited:
- WO-A2-2011/092295
- US-A1- 2007 059 306
- US-A1- 2010 189 722
- DATABASE BIOSIS [Online] BIOSCIENCES INFORMATION SERVICE, PHILADELPHIA, PA, US; October 2011 (2011-10), HEIDER KARL-HEINZ ET AL: "A novel Fc-engineered monoclonal antibody to CD37 with enhanced ADCC and high proapoptotic activity for treatment of B-cell malignancies", XP002693616, Database accession no. PREV201100741993 & BLOOD, vol. 118, no. 15, October 2011 (2011-10), pages 4159-4168, ISSN: 0006-4971(print), DOI: 10.1182/BLOOD-2011-04-351932

## Description

### Technical field of the invention

The present invention relates to radioimmunotherapy of hematologic cancer with a radiolabeled chimeric antibody with an unexpectedly high cytotoxicity as well as various applications of the antibodies.

### Background of the invention

The present invention relates to chimeric and humanized anti-CD37 antibodies as well as the production and applications hereof.

The present invention furthermore relates to immunotherapies and radioimmunotherapies that are based on B-cell depletion.

In particular, the present invention relates to anti-CD37 antibody molecules for use in such therapies, e.g. in the treatment of B cell malignancies and autoimmune conditions.

Immunotherapy using monoclonal antibodies (mAbs) has been emerging as a safe and selective method for the treatment of cancer and other diseases.

In particular, the role of monoclonal antibodies in therapies that are based on B-cell depletion, e.g. in the treatment of B-cell malignancies, has expanded since the introduction of rituximab, an antibody that is directed against the CD20 antigen on the B-cell surface.

The CD37 antigen is a cell surface antigen that has not been considered as a target for B cell malignancies to the same extent as the B-cell antigen CD20.

CD37, a member of the tetraspanin superfamily, is a heavily glycosylated cell surface molecule with four transmembrane domains and two extracellular loops. CD37 expression is observed in normal B-cells, non-Hodgkin's lymphoma (NHL), including mantle cell lymphoma (MCL), Burkitts Lymphoma (BL), small lymphocytic lymphoma (SLL) and follicular lymphoma (FL), marginal zone lymphoma (MZL), Diffuse large B-cell lymphoma (DLBCL), lymphoblastic lymphoma (LL), and chronic lymphoid leukemia (CLL).

This expression pattern makes CD37 an attractive target for antibody-mediated cancer therapy.

CD37 was first described in 1986 and characterized by the murine monoclonal antibody MB-1 (Link et al, 1986).

The physiological role of CD37 is unknown.

Binding of a CD37-specific mAb to cancer cells may trigger various mechanisms of action: First, after the antibody binds to the extracellular domain of the CD37 antigen, it may activate the complement cascade and lyse the targeted cell.

Second, an anti-CD37 antibody may mediate antibody-dependent cell-mediated cytotoxicity (ADCC) to the target cell, which occurs after the Fc portion of the bound antibody is recognized by appropriate receptors on cytotoxic cells of the immune system.

Third, the antibody may alter the ability of B-cells to respond to antigen or other stimuli.

Finally, anti-CD37 antibody may initiate programmed cell death
(apoptosis).

Anti-CD37 mAb MB-1 was evaluated in two radio-immunotherapy trials in B-NHL patients (B-cell non-Hodgkin's lymphoma; Press et al., 1989; Kaminski et al., 1992).

Others have also disclosed anti-CD37 mABs that show potential (e.g. WO 2009/019312 by Heider et al. and WO 2011/092295 by the present inventors) but there is still a long way to go before CD37 is proven the ideal alternative to CD20 for treating B-cell malignancies.

In conclusion, it has been shown that the CD37 antigen is frequently expressed on tumor cells in several human B-cell malignancies and on mature normal B-lymphocytes and that anti-CD37-based therapy may be a promising approach for treating B cell malignancies.

Although the anti-CD37 antibodies or antibody-like molecules described above (e.g. MB-1) have shown anti-tumor efficacy in B-cell malignancies and the potential to target CD37, there is a need for alternate anti-CD37 antibodies to improve therapies based on B-cell depletion.

Hence, an improved anti-CD37 antibody would be advantageous in the pursuit against new treatments against B-cell malignancies.

### Summary of the invention

An object of the present disclosure relates to a chimeric or humanized antibody derived from the mouse monoclonal antibody HH1.

In particular, it is an object of the present disclosure to provide a chimeric or humanized antibody.

One aspect of the present disclosure relates to an antibody molecule that binds to human CD37 and that is derived from a) a murine monoclonal antibody that is defined by i) a variable heavy chain comprising the amino acid sequence shown in SEQ ID NO: 1; and ii) a variable light chain comprising the amino acid sequence shown in SEQ ID NO :3, or from b) a non-human antibody recognizing the same epitope of human CD37 as the antibody defined in a) or recognizing an epitope that is close to or overlaps with said epitope; wherein said antibody molecule is a chimeric or a humanized antibody.

Another aspect of the present disclosure relates to a DNA molecule encoding the antibodies of the present invention.

Yet another aspect of the present disclosure relates to a host cell carrying one or more DNA molecules encoding the antibodies of the present invention.

Still another aspect of the present disclosure relates to a method for producing an antibody of the present invention, comprising transfecting a mammalian host cell with one or more vectors encoding the antibodies of the present invention, culturing the host cell and recovering and purifying the antibody molecule.

Another aspect of the present disclosure relates to a pharmaceutical composition comprising, as the active ingredient, one or more antibodies of the present invention, and a pharmaceutically acceptable carrier.

Another aspect of the present disclosure relates to a pharmaceutical composition for use in the treatment of B-cell malignancies.

Yet another aspect of the present disclosure relates to method for treating a patient suffering from a B-cell malignancy selected from the group consisting of B-cell non-Hodgkins lymphoma, B-cell chronic lymphocytic leukemia, hairy cell leukemia, lymphoplasmacytic lymphoma and multiple myeloma, comprising administering to said patient an effective amount of a pharmaceutical composition of the present invention.

Still another aspect of the present disclosure relates to a radioimmunoconjugate that binds human CD37 comprising a) an antibody of the present invention, b) a linker, and c) a radionuclide selected from the group consisting of ²¹¹At, ²¹³Bi, ²¹²Bi, ²¹²Pb, ²²⁵Ac, ²²⁷Th, ⁹⁰Y, ¹⁸⁶Re, ¹⁸⁸Re, ¹⁹⁹Au, ¹⁹⁴Ir, ¹⁶⁶Ho, ¹⁵⁹Gd, ¹⁵³Sm, ¹⁴⁹Pm, ¹⁴²Pr, ¹¹¹Ag, ¹⁰⁹Pd, ⁷⁷As, ⁶⁷Cu, ⁴⁷Sc, and ¹⁷⁷Lu.

An object of the present invention relates to a radioimmunoconjugate that binds human CD37 comprising a) an antibody molecule that binds to human CD37, and that is a chimeric antibody, defined by i) a variable heavy chain comprising an amino acid sequence encoded by the nucleic acid sequence shown in SEQ ID NO: 1; ii) a variable light chain comprising an amino acid sequence encoded by the nucleic acid sequence shown in SEQ ID NO:3, iii) constant heavy and light chains that are of human origin, wherein said constant heavy chain i) comprises amino acid sequences encoded by the nucleic acid sequences shown in SEQ ID NO:5, SEQ ID NO:6, and SEQ ID NO:7 and wherein said constant light chain ii) comprises an amino acid sequence encoded by the nucleic acid sequence shown in SEQ ID NO:9, b) a linker, and c) a ²¹²Pb radionuclide.

Another aspect of the present invention relates to a pharmaceutical composition comprising a radioimmunoconjugate as described above.

Another aspect of the present invention relates to the pharmaceutical as described above for use in the treatment of a B-cell malignancy selected from the group consisting of B-cell non-Hodgkins lymphoma, B-cell chronic lymphocytic leukemia, hairy cell leukemia, lymphoplasmacytic lymphoma and multiple myeloma.

A further aspect of the present invention relates to a method for treatment of a B-cell malignancy selected from the group consisiting of B-cell non-Hodgkins lymphoma, B-cell chronic lymphocytic leukemia, hairy cell leukemia, lymphoplasmacytic lymphoma and multiple myeloma, comprising administration of an effective amount of a pharmaceutical composition of the present invention.

Yet another aspect of the present invention relates to a kit for the production of the radioimmunoconjugate of the present invention, comprising two or more vials, wherein one vial contains a conjugate comprising a chelator linked to an antibody of the present invention; and a second vial contains a ²¹²Pb radionuclide.

Also an aspect is the use of the chimeric antibody to block CD37 antigen in normal tissues before using radiolabeled murine or chimeric HH1 for therapy.

### Brief description of the figures

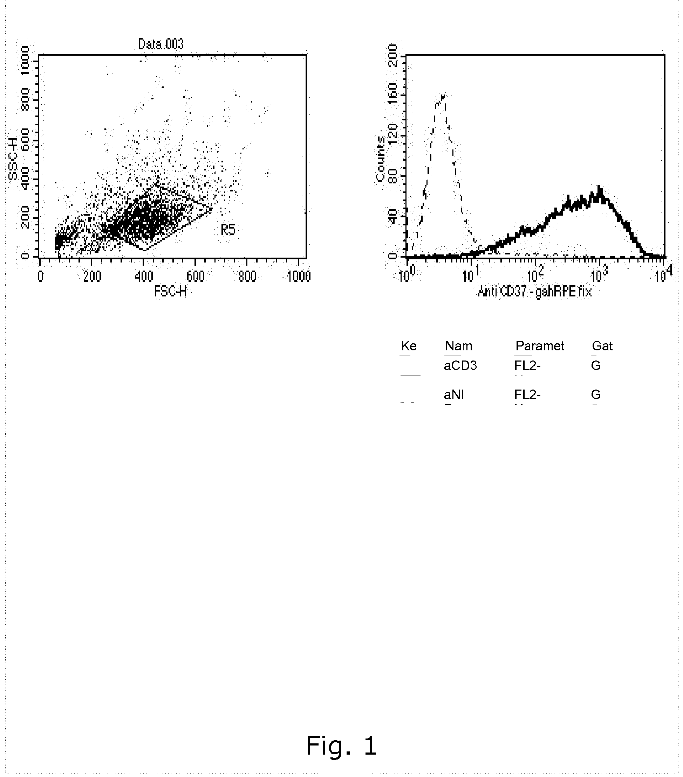

Figure 1 shows the flow cytometry dot plot for gating on the Daudi cells and the histogram for binding of anti-CD37 chHH1.1 (IgG1 isotype) and no binding of anti-NIP antibody (negative control). Figure 1 shows a significant antigen binding of chHH1.
Figure 2 shows the cellular binding of chHH1.1 against Daudi lymphoma cells. It shows a rapid and efficient binding to the target.
Figure 3 shows that chHH1 has a similar ADCC as rituximab despite fewer antigens and a signifcant internalization of CD37 in the Daudi lymphoma target cells.
Figure 4 shows ADCC with IL-2 stimulated PBMC as effector cells and Rec-1 cells as target cells at three different ratios of effector to target cells. Mean and SD of three individuals.
Figure 5 shows CDC with 10 % serum from three individuals on Rec-1 cells labeled with murine HH1, rituximab, chHH1.1 or a combination of rituximab and chHH1.1.
Figure 6 shows biodistribution (% I.D./g) of ¹²⁵I-chHH1.3 in female nude mice 24 and 48 hours after injection.
Figure 7 shows percentage specific lysis by ADCC in Daudi cells with NK cells as effector. The blood was taken from two individuals and one experiment was done with a ratio of 10 NK cells to 1 Daudi cells and the other experiment with a 15:1 ratio.

The present invention will now be described in more detail in the following.

### Detailed description of the invention

The present disclosure relates to humanized or chimeric antibodies derived from the mouse monoclonal antibody HH1.

Humanized or chimeric antibodies are antibodies from non-human species whose protein sequences have been modified to increase their similarity to antibody variants produced naturally in humans.

The process of "humanization" is usually applied to monoclonal antibodies developed for administration to humans.

Humanization can be necessary when the process of developing a specific antibody involves generation in a non-human immune system (such as that in mice).

The protein sequences of antibodies produced in this way are partially distinct from homologous antibodies occurring naturally in humans, and are therefore potentially immunogenic when administered to human patients.

Not all monoclonal antibodies designed for human administration need be humanized since many therapies are short-term interventions.

Humanization is usually seen as distinct from the creation of a mouse-human antibody chimera.

So, although the creation of an antibody chimera is normally undertaken to achieve a more human-like antibody (by substituting the mouse Fc region of the antibody with that from human) simple chimeras of this type are not usually referred to as humanized.

Rather, the protein sequence of a humanized antibody is essentially identical to that of a human variant, despite the non-human origin of some of its complementarity determining region (CDR) segments responsible for the ability of the antibody to bind to its target antigen.

However, in the present context the term chimeric antibody and humanized antibody is used interchangeably as referring to an antibody that has been genetically engineered and is derived from the mouse monoclonal antibody HH1.

Chimeric or humanized antibodies derived from the monoclonal antibody HH1

The immunoglobulin heavy chain (IgH) is the large polypeptide subunit of an antibody (immunoglobulin).

A typical antibody is composed of two immunoglobulin (Ig) heavy chains and two Ig light chains.

Several different types of heavy chain exist that define the class or isotype of an antibody.

These heavy chain types vary between different animals.

The immunoglobulin light chain is the small polypeptide subunit of an antibody (immunoglobulin).

There are two types of light chain in humans (as in other mammals), kappa (κ) chain, encoded by the immunoglobulin kappa locus on chromosome 2 and the lambda (λ) chain, encoded by the immunoglobulin lambda locus on chromosome 22.

Antibodies are produced by B lymphocytes, each expressing only one class of light chain.

Once set, light chain class remains fixed for the life of the B lymphocyte.

In a healthy individual, the total kappa to lambda ratio is roughly 2:1 in serum (measuring intact whole antibodies) or 1:1.5 if measuring free light chains, with a highly divergent ratio indicative of neoplasm.

The exact normal ratio of kappa to lambda ranges from 0.26 to 1.65.

Both the kappa and the lambda chains can increase proportionately, maintaining a normal ratio.

Both variable and constant chains in a chimeric or humanized antibody derived from the mouse monoclonal antibody HH1 can differ from known sequences.

Examples of such variations are clear from the present disclosure and include selection of constant chains, genetic variation of variable chains and variations of the Fc domain in order to modulate of effector functions.

The present inventors have genetically engineered chimeric, humanized antibodies derived from the mouse monoclonal antibody HH1.

These antibodies show a promising effect in the search for optimal treatment of B-cell related malignancies.

These effects are shown in the experiments of the present disclosure.

Thus, an aspect of the present disclosure relates to an antibody molecule that binds to human CD37 and that is derived from a) a murine monoclonal antibody that is defined by i) a variable heavy chain comprising the amino acid sequence shown in SEQ ID NO: 1; and ii) a variable light chain comprising the amino acid sequence shown in SEQ ID NO :3, or from b) a non-human antibody recognizing the same epitope of human CD37 as the antibody defined in a) or recognizing an epitope that is close to or overlaps with said epitope; wherein said antibody molecule is a chimeric or a humanized antibody.

In an embodiment of the present disclosure is the chimeric antibody defined by i) a variable heavy chain comprising the amino acid sequence shown in SEQ ID NO: 1; ii) a variable light chain comprising the amino acid sequence shown in SEQ ID NO:3, iii) constant heavy and light chains that are of human origin.

In another embodiment of the present disclosure is the chimeric antibody defined by i) the constant heavy chain is selected from the group consisting of IgG1, IgG2, IgG3 and IgG4 chain, and ii) the constant light chain is a kappa or a lambda chain.

In a further embodiment of the present disclosure is the constant heavy chain defined by i) comprising the amino acid sequence shown in SEQ ID NO:5, SEQ ID NO:6 and/or SEQ ID NO:7 and wherein the constant light chain ii) comprises the amino acid sequence shown in SEQ ID NO:9.

In a further embodiment of the present disclosure is the constant heavy chain defined by i) comprising the amino acid sequence shown in SEQ ID NO:11 and/or SEQ ID NO:14 and wherein the constant light chain ii) comprises the amino acid sequence shown in SEQ ID NO:13.

DNA chHH1.3 Fc sequence with mutation full length heavy chain can be seen as SEQ ID NO: 10.

Amino acid chHH1.3 Fc sequence with mutation full length light chain can be seen as SEQ ID NO: 11.

DNA chHH1.3 Fc sequence with mutation full length heavy chain can be seen as SEQ ID NO: 12.

Amino acid chHH1.3 Fc sequence with mutation full length light chain can be seen as SEQ ID NO:13.

chHH1.3 Fc sequence without mutation (constant region) can be seen as SEQ ID NO: 14.

### Nucleic acid molecules encoding the antibodies of the present disclosure

The humanization processes takes advantage of the fact that production of monoclonal antibodies can be accomplished using recombinant DNA to create constructs capable of expression in mammalian cell culture.

That is, gene segments capable of producing antibodies are isolated and cloned into cells that can be grown in a tank such that antibody proteins produced from the DNA of the cloned genes can be harvested *en masse.*

The step involving recombinant DNA provides an intervention point that can be readily exploited to alter the protein sequence of the expressed antibody.

The alterations to antibody structure that are achieved in the humanization process are therefore all effectuated through techniques at the DNA level.

Thus, an aspect of the present disclosure relates to a DNA molecule encoding the humanized or chimeric antibodies of the present invention.

In one embodiment of the present disclosure encodes the DNA molecule a region encoding the variable heavy chain of a humanized or chimeric antibody of the present invention.

In another embodiment of the present disclosure is this variable heavy chain encoding region fused to a region encoding a constant heavy chain of human origin.

Such human constant heavy chain can be selected from the group consisting of IgG1, IgG2, IgG3 and IgG4.

In one embodiment of the present disclosure is the IgG1 encoded by the sequence shown in SEQ ID NO:5, SEQ ID NO:6 and/or SEQ ID NO:7

In one embodiment of the present disclosure is the IgG3 heavy chain encoded by the sequence shown in SEQ ID NO:10.

In one embodiment of the present disclosure is the IgG3 light chain encoded by the sequence shown in SEQ ID NO:12.

In one embodiment of the present disclosure is the IgG3 with H435 substitution mutation encoded by the sequence shown in SEQ ID NO:12.

In a further embodiment of the present disclosure comprises the human constant heavy chain one or more substitutions in the Fc region.

Another embodiment of the present disclosure relates to a DNA molecule comprising a region encoding the variable light chain of the chimeric or humanized antibody of the present invention.

Such variable light chain encoding region may be fused to a region encoding a constant light chain of human origin.

The constant light chain may be a kappa or a lambda chain.

In one embodiment of the present disclosure is the kappa light chain encoded by a sequence shown in SEQ ID NO:9.

The DNA molecules can be constructed and optimized for expression in a target cell.

An expression vector, otherwise known as an expression construct, is generally a plasmid that is used to introduce a specific gene (in the present context the DNA molecule of the present invention) into a target cell.

Once the expression vector is inside the cell, the protein that is encoded by the gene is produced by the cellular-transcription and translation machinery ribosomal complexes.

The plasmid is frequently engineered to contain regulatory sequences that act as enhancer and promoter regions and lead to efficient transcription of the gene carried on the expression vector.

The goal of a well-designed expression vector is the production of large amounts of stable messenger RNA, and therefore proteins.

Expression vectors are basic tools for biotechnology and the production of proteins such as insulin that are important for medical treatments of specific diseases like diabetes.

After expression of the gene product, the purification of the protein is required; but since the vector is introduced to a host cell, the protein of interest should be purified from the proteins of the host cell.

Therefore, to make the purification process easy, the cloned gene should have a tag. This tag could be histidine (His) tag or any other marker peptide.

Thus, an aspect of the present disclosure therefore relates to an expression vector comprising a DNA molecule as described above.

### Cells comprising and expressing the antibodies of the present disclosure

The above described DNA molecules or expression vectors can be introduced into host cells.

Such cells can through hybridoma technology become immortalized cells that produce the chimeric or humanized antibodies.

Hybridoma technology is a technology of forming hybrid cell lines (called hybridomas) by fusing a specific antibody-producing B cell with a myeloma (B cell cancer) cell that is selected for its ability to grow in tissue culture and for an absence of antibody chain synthesis.

The antibodies produced by the hybridoma are all of a single specificity and are therefore monoclonal antibodies (in contrast to polyclonal antibodies).

Thus, one aspect of the present disclosure relates to a host cell carrying one or more vectors or DNA molecules of the present invention.

One embodiment of the present invention relates to a host cell carrying an expression vector comprising a DNA molecule encoding the variable heavy chain of HH1, and a second expression vector comprising a DNA molecule encoding the variable light chain of the present invention.

In one embodiment of the present disclosure is the host cell a mammalian cell.

In another embodiment of the present disclosure is the cell a hybridoma cell.

### Methods for producing the antibodies of the present disclosure

The chimeric or humanized antibodies of the present disclosure can be produced by several methods.

One method for producing such antibodies comprises transfecting a mammalian host cell with one or more vectors of the present invention, culturing the host cell and recovering and purifying the antibody molecule.

Another method for producing such antibodies comprising construction of hybridoma cells that produce the chimeric or humanized antibodies of the present invention.

### Sequence identity

As commonly defined "identity" is here defined as sequence identity between genes or proteins at the nucleotide or amino acid level, respectively.

Thus, in the present context "sequence identity" is a measure of identity between proteins at the amino acid level and a measure of identity between nucleic acids at nucleotide level.
The protein sequence identity may be determined by comparing the amino acid sequence in a given position in each sequence when the sequences are aligned.

Similarly, the nucleic acid sequence identity may be determined by comparing the nucleotide sequence in a given position in each sequence when the sequences are aligned.

To determine the percent identity of two nucleic acid sequences or of two amino acids, the sequences are aligned for optimal comparison purposes (e.g., gaps may be introduced in the sequence of a first amino acid or nucleic acid sequence for optimal alignment with a second amino or nucleic acid sequence). The amino acid residues or nucleotides at corresponding amino acid positions or nucleotide positions are then compared.

When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity = # of identical positions/total # of positions (e.g., overlapping positions) x 100). In one embodiment the two sequences are the same length.

One may manually align the sequences and count the number of identical nucleic acids or amino acids. Alternatively, alignment of two sequences for the determination of percent identity may be accomplished using a mathematical algorithm. Such an algorithm is incorporated into the NBLAST and XBLAST programs. BLAST nucleotide searches may be performed with the NBLAST program, score = 100, wordlength = 12, to obtain nucleotide sequences homologous to a nucleic acid molecules of the invention. BLAST protein searches may be performed with the XBLAST program, score = 50, wordlength = 3 to obtain amino acid sequences homologous to a protein molecule of the invention.

To obtain gapped alignments for comparison purposes, Gapped BLAST may be utilised. Alternatively, PSI-Blast may be used to perform an iterated search which detects distant relationships between molecules. When utilising the NBLAST, XBLAST, and Gapped BLAST programs, the default parameters of the respective programs may be used. See http://www.ncbi.nlm.nih.gov. Alternatively, sequence identity may be calculated after the sequences have been aligned e.g. by the BLAST program in the EMBL database (www.ncbi.nlm.gov/cgi-bin/BLAST).

Generally, the default settings with respect to e.g. "scoring matrix" and "gap penalty" may be used for alignment. In the context of the present invention, the BLASTN and PSI BLAST default settings may be advantageous.

The percent identity between two sequences may be determined using techniques similar to those described above, with or without allowing gaps. In calculating percent identity, only exact matches are counted.

An embodiment the disclosure relates to an isolated nucleic acid comprising a nucleic acid sequence sharing 80 % sequence identity with the HH1 antibody VH sequence (SEQ ID NO: 2) and/or VL sequence (SEQ ID NO: 4).

An embodiment the disclosure relates to an isolated nucleic acid comprising a nucleic acid sequence with the HH1 antibody VH sequence (SEQ ID NO: 2) and/or VL sequence (SEQ ID NO: 4).

In another embodiment of the disclosure the isolated nucleic acid comprises a nucleic acid sequence sharing at least 90 % sequence identity with the HH1 antibody VH sequence (SEQ ID NO: 2) and/or VL sequence (SEQ ID NO: 4), such as 90 % identity, 91 % identity, 92 % identity, 93 % identity, 94 % identity, 95 % identity, 96 % identity, 97 % identity, 98 % identity, or 99 % identity.

Another embodiment of the disclosure relates to an antibody comprising a polypeptide sequence sharing 80 % sequence identity with the HH1 antibody VH sequence (SEQ ID NO: 1) and/or VL sequence (SEQ ID NO: 3).

Another embodiment of the disclosure relates to an antibody comprising a polypeptide sequence with the HH1 antibody VH sequence (SEQ ID NO: 1) and/or VL sequence (SEQ ID NO: 3).

In another embodiment of the present disclosure, the antibody comprises a polypeptide sequence sharing at least 90 % sequence identity with the HH1 antibody VH sequence (SEQ ID NO: 1) and/or VL sequence (SEQ ID NO: 3), such as 90 % identity, 91 % identity, 92 % identity, 93 % identity, 94 % identity, 95 % identity, 96 % identity, 97 % identity, 98 % identity, or 99 % identity.

In a preferred embodiment of the present disclosure are these antibodies a chimeric or humanized antibody derived from the monoclonal antibody HH1.

### Genetic variation

Genetic variation is caused by variation in the order of bases in the nucleotides in genes. This variation cause mutations in the genes and subsequently in the proteins that such genes encode.

These mutations can be either sense or missense mutations or substitutions.

An embodiment of the present disclosure relates to the isolated nucleic acid sequence of the HH1 monoclonal antibody VH chain (SEQ ID NO: 2) and/or VL chain (SEQ ID NO: 4) that comprises at least 50, such as 20, such as 10, such as 5, such as 4, such as 3, such as 2, such as 1 sense mutations.

Another embodiment of the present disclosure relates to the isolated nucleic acid sequence of the HH1 monoclonal antibody VH chain (SEQ ID NO: 2) and/or VL chain (SEQ ID NO: 4) that comprises 0-50, such as 1-50, such as 0-20, such as 1-20, such as 0-10, such as 1-10, such as 0-5, such as 1-5, such as 3, such as 1 sense mutations.

A missense mutation (a type of non-synonymous mutation) is a point mutation in which a single nucleotide is changed, resulting in a codon that codes for a different amino acid (mutations that change an amino acid to a stop codon are considered nonsense mutations, rather than missense mutations). A missense mutation can render the resulting protein non-functional.

However, not all missense mutations lead to appreciable protein changes. An amino acid may be replaced by an amino acid of very similar chemical properties, in which case, the protein may still function normally; this is termed a neutral, "quiet", or conservative mutation.

Alternatively, the amino acid substitution could occur in a region of the protein which does not significantly affect the protein secondary structure or function. When an amino acid may be encoded by more than one codon (so-called "degenerate coding") a mutation in a codon may not produce any change in translation; this would be a synonymous mutation (a form of silent mutation) and not a missense mutation.

An embodiment of the present disclosure relates to an antibody comprising a polypeptide sequence of the HH1 monoclonal antibody VH chain (SEQ ID NO: 2) and/or VL chain (SEQ ID NO: 4) that comprises at least 50, such as 20, such as 10, such as 5, such as 4, such as 3, such as 2, such as 1 missense mutations.

An embodiment of the present disclosure relates to an antibody comprising a polypeptide sequence of the HH1 monoclonal antibody VH chain (SEQ ID NO: 2) and/or VL chain (SEQ ID NO: 4) that comprises 0-50, such as 1-50, such as 0-20, such as 1-20, such as 0-10, such as 1-10, such as 0-5, such as 1-5, such as 3, such as 1 missense mutations.

A conservative substitution is a substitution of one amino acid with another with generally similar properties such that the overall functioning is likely not to be seriously affected.

In another embodiment of the present invention are the missense mutations conservative mutations or substitutions.

A further embodiment of the present disclosure relates to an isolated nucleic acid sequence or a polypeptide sequence with 80% sequence identity to the variable heavy chain (SEQ ID NO: 1) and/or variable light chain (SEQ ID NO: 3) sequences af HH1, wherein the sequence variation is conservative substitutions.

In another embodiment of the present disclosure is the sequence identity 80% identity, such as 90% identity, 91 % identity, 92 % identity, 93 % identity, 94 % identity, 95 % identity, 96 % identity, 97 % identity, 98 % identity, or 99 % identity and the sequence variation is conservative substitutions.

In order to improve the radiolabeling step it may be beneficial to introduce extra lysine into e.g., the Fc portion of the chimeric or humanized antibody of the present invention.

This could reduce the probability of attaching lysine binding chelators into the antigen combining sites at the antibody, thereby reducing the risk of compromizing immunoreactivity during radiolabeling.

Methods for introducing lysine into e.g. the Fc portion of HH1 is known in the art e.g. from Hemminki et al., 1995.

An embodiment of the present disclosure relates to the radioimmunoconjugate of the present invention which has been modified by 10 Lys in the Fc portion of HH1, such as 8 Lys, such as 6 Lys, such as 5 Lys, such as 4 Lys, such as 3 Lys, such as 2 Lys, such as 1 Lys.

Other variations of the Fc portion of the antibodies of the present disclosure can be chosen in order to optimize or modulate one or more effector functions.

These modulations of effector functions are made e.g. to increase in antibody-dependent cell-mediated cytotoxicity (ADCC).

Such variations of the Fc portion are known in the art.

Thus, one aspect of the present disclosure relates to a antibody of the present disclosure that has one or more mutations in the Fc domain that modulate one or more effector functions.

### Immonoconjugates

Immunoconjugates are antibodies conjugated (joined) to a second molecule, usually a toxin, radioisotope or label.

Such immunoconjugates of chimeric and humanized HH1 are all aspects of the present disclosure.

One type is the chimeric and humanized HH1 of the present invention connected to or associated with a chelating linker.

Chelating linkers are discussed in the below section regarding radioimmunoconjugates and the chelating linkers described therein are therefore all considered useful for immunoconjugates comprising chimeric and humanized HH1 of the present invention connected to or associated with a chelating linker

### Radioimmunoconjugates

An aspect of the present disclosure relates to a radioimmunoconjugate that binds human CD37 comprising a chimeric or humanized antibody derived from the monoclonal antibody HH1 according to the present invention, a linker, and a radionuclide selected from the group consisting of ²¹¹At, ²¹³Bi, ²¹²Bi, ²¹²Pb, ²²⁵Ac, ²²⁷Th, ⁹⁰Y, ¹⁸⁶Re, ¹⁸⁸Re, ¹⁹⁹Au, ¹⁹⁴Ir, ¹⁶⁶Ho, ¹⁵⁹Gd, ¹⁵³Sm, ¹⁴⁹Pm, ¹⁴²Pr, ¹¹¹Ag, ¹⁰⁹Pd, ⁷⁷As, ⁶⁷Cu, ⁴⁷Sc, and ¹⁷⁷Lu.

An object of the present invention relates to a radioimmunoconjugate that binds human CD37 comprising a) an antibody molecule that binds to human CD37, and that is a chimeric antibody, defined by i) a variable heavy chain comprising an amino acid sequence encoded by the nucleic acid sequence shown in SEQ ID NO: 1; ii) a variable light chain comprising an amino acid sequence encoded by the nucleic acid sequence shown in SEQ ID NO:3, iii) constant heavy and light chains that are of human origin, wherein said constant heavy chain i) comprises amino acid sequences encoded by the nucleic acid sequences shown in SEQ ID NO:5, SEQ ID NO:6, and SEQ ID NO:7 and wherein said constant light chain ii) comprises an amino acid sequence encoded by the nucleic acid sequence shown in SEQ ID NO:9, b) a linker, and c) a ²¹²Pb radionuclide.

In an embodiment of the present invention the linker is a chelating linker.

In another embodiment of the present disclosure is the radionuclide selected from the group consisting of ¹⁷⁷Lu, ²²⁵Ac, ²²⁷Th and ⁹⁰Y.

In another embodiment of the present disclosure the radionuclide is ¹⁷⁷Lu.

The radionuclide is ²¹²Pb.

In yet another embodiment the radionuclide is another beta-emitter or an alpha-emitter.

The radionuclide may be attached to the antibody by first reacting a bifunctional chelator, e.g., p-SCN-bn-DOTA (Macrocyclics, Tx, USA), with the antibody, followed by purification to remove unconjugated chelator, and then reaction of the chelator antibody conjugate with the radionuclide, followed by purification to remove any unconjugated radionuclide.

Alternatively, the chelator and the radionuclide can be combined firstly and subsequently conjugated to the antibody.

Chelating linkers like, e.g., p-SCN-bn-DOTA, can be used for conjugating other metal radionuclides to HH1 derived antibodies in similar fashion to that described for ¹⁷⁷Lu.

Any type of linker with sufficient complexing ability and a functional group allowing direct or indirect conjugation to a protein or a peptide could be used. Examples of such linkers are described in the literature (e.g. Brechbiel, 2008; Liu, 2008). Some useful examples are bifunctional cyclic chelators like p-SCN-bn-DOTA, DOTA-NHS-ester, p-SCN-Bn-TCMC; bifunctional linear chelators like p-SCN-Bn-DTPA and CHX-A"-DTPA.

The radionuclides in the present invention will preferably be conjugated to a targeting molecule by using bifunctional chelators.

These could be cyclic, linear or branched chelators. Particular reference may be made to the polyaminopolyacid chelators which comprise a linear, cyclic or branched polyazaalkane backbone with acidic (e.g. carboxyalkyl) groups attached at backbone nitrogens.

Examples of suitable chelators include DOTA derivatives such as p-isothiocyanatobenzyl-1,4,7,10-tetraazacyclododecane-1,4,7,10-tetraacetic acid (p-SCN-Bz-DOTA) or S-2-(4-Isothiocyanatobenzyl)-1,4,7,10-tetra(2-carbamoylmethyl)cyclododecane and DTPA derivatives such as p-isothiocyanatobenzyl-diethylenetriaminepentaacetic acid (p-SCN-Bz-DTPA), the first being cyclic chelators, the latter linear chelators.

Metallation of the complexing moiety may be performed before or after conjugation of the complexing moiety to the targeting moiety.

The radiolabeling procedure will in general be more convenient in terms of time used etc if the chelator is conjugated to the antibody before the radiolabeling takes place.

The principles of preparing radiolabeled conjugates using chelators attached to antibodies are described broader in e.g. Liu, 2008.

Thus, HH1 derived chimieric or humanized antibodies can be used to prepare radioimmunoconjugates with differences in radiation properties and effective half-lives.

For example anti-CD37 radioimmunoconjugate consisting of a chimeric or humanized antibody derived from the monoclonal antibody HH1 according to the present invention, a chelating linker and a beta or alpha emitting radionuclide including, but not limited to ¹⁷⁷Lu, ²¹¹At, ²¹³Bi, ²¹²Bi, ²¹²Pb, ²²⁵Ac, ²²⁷Th, ⁹⁰Y, ¹⁸⁶Re, ¹⁸⁸Re, ¹⁹⁹Au, ¹⁹⁴Ir, ¹⁶⁶Ho, ¹⁵⁹Gd, ¹⁵³Sm, ¹⁴⁹Pm, ¹⁴²Pr, ¹¹¹Ag, ¹⁰⁹Pd, ⁷⁷As, ⁶⁷Cu, ⁴⁷Sc can be prepared and used for preparing pharmaceutical preparations and used in therapeutic applications.

### Immunotoxins

An immunotoxin is a human-made protein that consists of a targeting portion linked to a toxin. When the protein binds to that cell, it is taken in through endocytosis, and the toxin kills the cell.

They are usually used for the treatment of some kinds of cancer and a few viral infections.

These proteins are usually made of a modified antibody or antibody fragment, attached to a fragment of a toxin.

The targeting portion is composed of the Fv portion of an antibody that targets a specific cell type.

The toxin is usually a cytotoxic protein derived from a bacterial or plant protein, from which the natural binding domain has been removed so that the Fv directs the toxin to the antigen on the target cell.

In another embodiment of the disclosure the toxin is a chemotherapeutic molecule, including, but not limited to alkylating agents (cisplatin, carboplatin, oxaliplatin, mechlorethamine, cyclophosphamide, chlorambucil, ifosfamide), antimetabolites (azathioprine, mercaptopurine, pyrimidines), alkaloids (vincristine, vinblastine, cinorelbine, vindesine, paclitaxel, docetaxel, etoposide, teniposide), topoisomerase inhibitors (irinotecan, topotecan, amascrine, etoposide, teniposide) and cytotoxic antibiotics (actinomycin, doxorubicin, daunorubicin, calrubicin, idarubicin, epirubicin, bleomycin, plicamycin, mitomycin).

In one embodiment of the disclosure doxorubicin is conjugated to the antibody via the cross-linker SMCC-hydrazide (4-[N-maleimidomethyl]cyclohexane-1 carboxylhydrazide).

The immunotoxin works by the antibody (or other targeting moiety) binding to an antigen on the target cell followed by toxin that enters and kills the cell.

Thus, an aspect of the present disclosure relates to an immunotoxin that comprises the antibody of the present invention or a fragment hereof.

### Pharmaceutical compositions

Antibodies are usually applied in the treatment of diseases formulated in pharmaceutical compositions.

Such compositions are optimized for parameters such as physiological tolerance and shelf-life.

Thus, relates and aspect of the present disclosure to a pharmaceutical composition comprisingone or more anti-CD37 radioimmunoconjugate molecules of the present invention (i.e. the chimeric), and a pharmaceutically acceptable carrier.

An embodiment of the present invention relates to a pharmaceutical composition as described above, further comprising one or more additional therapeutic agents.

In one embodiment of the present invention are said one or more additional therapeutic agents are selected from agents that target a B-cell antigen other than CD37.

Such antigen may be the B-cell antigen CD20.

In another embodiment of the present invention are said one or more additional therapeutic agents selected from agents that induce apoptosis.

A radioimmunotherapeutic product based on chimeric or humanized HH1 would typically be provided as a pharmaceutical composition consisting of a radionuclide, according to the description above, linked via a chelator to the chimeric or humanized antibody HH1 dissolved in a buffer solution, which to a substantial degree maintain the chemical integrity of the radioimmunoconjugate and is being physiologically acceptable for infusion into patients.

Thus, an aspect of the present invention relates to a pharmaceutical composition comprising a radioimmunoconjugate of the present invention, and a pharmaceutically acceptable carrier and/or excipient.

Acceptable pharmaceutical carriers include but are not limited to non-toxic buffers, fillers, isotonic solutions, etc. More specifically, the pharmaceutical carrier can be but are not limited to normal saline (0.9 %), half-normal saline, Ringer's lactate, 5 % Dextrose, 3.3 % Dextrose/0.3 % Saline. The physiologically acceptable carrier can contain a radiolytic stabilizer, e.g., ascorbic acid, which protect the integrity of the radiopharmaceutical during storage and shipment.

One embodiment of the present invention comprises the pharmaceutical composition of the present invention and one or more additional antibodies or radioimmunoconjugates. Antibodies include but are not limited to Rituximab, Epratuzumab, L19, F8, F16, Galiximab, Toralizumab, Alemtuzumab, Ofatumumab, Veltuzumab, Afutuzumab, Tositumomab, Reditux, Ibritumomab, K7153A, 37.1 and HH1.

Radioimmunoconjugates include but are not limited to Zevalin, Bexxar and Betalutin.

In another embodiment of the present invention one or more additional antibodies or radioimmunoconjugates target CD20. Antibodies include but are not limited to Rituximab, Veltuzumab, Ofatumumab, Afutuzumab, Tositumomab, Reditux and Ibritumomab. Radioimmunoconjugates include but are not limited to Zevalin and Bexxar.

A further embodiment of the present invention relates to a pharmaceutical composition of the present invention for treating B-cell malignant cells expressing the CD37 antigen.

In an embodiment of the present invention the pharmaceutical composition is for treatment of a B-cell malignancy selected from the group consisiting of B-cell non-Hodgkins lymphoma, B-cell chronic lymphocytic leukemia, hairy cell leukemia, small lymphoblastic lymphoma and multiple myeloma.

### Treatment

Therapeutic use of a pharmaceutical solution according to the present invention may be for treatment against malignant cells expressing the CD37 antigen, including but not limited to a B-cell malignancy selected from the group consisiting of B-cell non-Hodgkins lymphoma, B-cell chronic lymphocytic leukemia, hairy cell leukemia, lymphoplasmacytic lymphoma and multiple myeloma.

Other uses could be treatment of autoimmune diseases and treatment of transplantation related effects.

The therapy could be based on, but are not limited to, beta-particle-radiation or alpha-particle-radiation or a combination of these.

The therapy could be administered either as a monotherapy or in combination with other therapies, preferentially standard treatments. Such other therapies may be pretreatment, surgery, chemotherapy (including doxorubicin, vinblastin and gemcitabine), immunotherapy, photodynamic therapy, proteasome inhibitor (including bortezomib), histone deacetylase inhibitors (including vorinostat and suberoylanilide hydroxamic acid), vitamin D3 and vitamin D3 analogs, cell cycle checkpoint inhibitors (including UCN-01 and 2-(4-(4-Chlorophenoxy)phenyl)-1H-benzimidazole-5-carboxamide), hypoxic cell radiosensitizers (including metronidazole and misonidazole), apoptosis inducers (including withaferin A) radiosensitizers, radioimmunotherapy or a combination of two or more of these.

By administered is meant intravenous infusion or intravenous injection. More specifically, the radioimmunoconjugate of the present invention can be administered directly in a vein by a peripheral cannula connected to a drip chamber that prevents air embolism and allows an estimate of flow rate into the patient.

In one embodiment the radioimmunoconjugate can be administered in a repeated fashion.

In another embodiment of the present invention the radioimmunoconjugate could be administered in a repeated fashion but with different radionuclides, e.g., beta-radioimmunotherapy could be followed by alpha-radioimmunotherapy or vice versa.

An aspect of the present invention relates to the use of the radioimmunoconjugate of the present invention for the treatment of B-cell malignancies.

An embodiment of the present invention relates to the use of the radioimmunoconjugate of the present invention administered in combination with or in addition to other therapy.

In an embodiment of the present invention the other therapies is selected from pretreatment, chemotherapy, monoclonal antibody therapy, surgery, radiotherapy, and/or photodynamic therapy.

In another embodiment of the present invention the other therapies are bone marrow transplantation or stem cell transplantation and/or therapy.

Another embodiment of the present invention comprises therapeutic pretreatment using anti-CD20 and/or anti-CD37 monoclonal antibody prior to the treatment with the radioimmunoconjugate of the present invention.

In an embodiment of the present invention is the pretreatment done by administering the chimeric antibody of the present invention followed by treatment by radioimmunoconjugates of the chimeric antibody of HH1 or radioimmunoconjugates of the antibody HH1.

An aspect of the present disclosure relates to a method for treatment of a B-cell malignancy selected from the group consisiting of B-cell non-Hodgkins lymphoma, B-cell chronic lymphocytic leukemia, hairy cell leukemia, lymphoplasmacytic lymphoma and multiple myeloma, comprising administration of an effective amount of the pharmaceutical composition of the present invention.

In one embodiment of the present invention are the uses and methods of treatment of the present invention performed *in vitro* or *ex vivo.*

In an embodiment of the present invention the antibody dosing is 1-1000 mg per patient, more preferably 5-50 mg per patient, and ¹⁷⁷Lu amounting to 1 - 200 MBq/kg, more preferably 10-100 MBq/kg of bodyweight.

The pharmaceutical compositions of the present invention comprising the chimeric or humanized antibody of the present invention can be used in depleting B cells that express CD37 on their surface.

Such pharmaceutical compositions can be used in the treatment of B-cell malignancies.

These B-cell malignancies may be selected from the group consisting of B-cell non-Hodgkins lymphoma, B-cell chronic lymphocytic leukemia, hairy cell leukemia, lymphoplasmacytic lymphoma and multiple myeloma.

An embodiment of the present disclosure relates to a pharmaceutical composition comprising the chimeric or humanized antibody of the present invention for use in the treatment of autoimmune or inflammatory diseases that involve B-cells in their pathology.

Another embodiment of the present disclosure relates to a method of depleting CD37 expressing B-cells from a population of cells, comprising administering to said population of cells an antibody molecule of the present invention a pharmaceutical composition containing such antibody molecule.

Yet another embodiment of the present invention relates to a method for treating a patient suffering from a B-cell malignancy selected from the group consisiting of B-cell non-Hodgkins lymphoma, B-cell chronic lymphocytic leukemia and multiple myeloma, comprising administering to said patient an effective amount of a pharmaceutical composition comprising a radioimmunoconjugate of the present invention.

In a special embodiment a chimeric or humanized HH1 is administered to a patient, prior to therapy with a radiolabeled or an immuntoxin version of murine, chimeric or humanized HH1, to block normal tissue cells and improve tumor uptake.

The dosing should be sufficient to block normal tissue uptake but not excessive as this would reduce the tumor uptake. E.g. the chimeric or humanized HH1 should be given in a dosage between 0,5 mg and 1 g per patient.

It could be given e.g. one week before and /or 1-5 hours before the administration of the radioimmunoconjugate.

### Kits

An aspect of the present invention relates to a kit for the production of the radioimmunoconjugate of the present invention comprising two or more vials, wherein one vial contains a conjugate comprising a chelator linked to a murine monoclonal antibody HH1; and a second vial contains a ²¹²Pb radionuclide.

A kit may require some procedures to be performed, e.g., radiolabeling and/or purification to take place before infusion.

An embodiment of the present invention relates to a kit of the present invention, wherein the content of one or several of the vials are either lyophilized or in a solution.

By mixing the contents of the two vials to generate the radioimmunoconjugate the final product will appear. Thus, in another embodiment of the present invention the radioimmunoconjugate is generated by mixing the content of the two vials.

This product may need purification prior to use.

It should be noted that embodiments and features described in the context of one of the aspects of the present invention also apply to the other aspects of the invention.

The invention will now be described in further details in the following non-limiting examples.

### Examples

### Example 1 Generation of chimeric antibody

A chimeric version of the HH1 antibody was made using expression vectors for the V-region gene.

The vectors accept VH and VL chain genes obtained by RT-PCR.

The V-genes were then sequenced and new specific primers were designed to amplify the V-genes before they were cloned into the pLNOH2 vector containing the human IgG antibody constant region genes (Cγ3, Cγ1 and CH1γ3).

The pLNOH2 vector including the constant regions IgG1CH1 (SEQ ID NO. 5), IgG1CH2 (SEQ ID NO. 6), IgG1CH3 (SEQ ID NO. 7) is shown in SEQ ID NO. 8.

The heavy chain and the light chain of the vectors pLNOH2 (SEQ ID NO. 5) and pLNOκ were combined to make the combi vector pLNOH2γ1/κ αCD37. The complete light chain gene (SEQ ID NO:9), the CMV promoter and polyA signal of pLNOκ αCD37 were subcloned over to pLNOH2 hIgG1 αCD37.

In the combi vector, the heavy and light chains are expressed from their own CMV promoter.

### Identification of constant domain

The constant domains in the pLNOH2 sequence were identified from the sequence of a human IgG1 in the IMGT database (accession number: Z17370).

The gene and protein sequence of the variable regions of the chHH1 anti-CD37 antibody is as follows:
VH αCD37 (aminoacid sequence: SEQ ID NO: 1 and nucleic acid sequence: SEQ ID NO: 2)
VL αCD37 (aminoacid sequence: SEQ ID NO: 3 and nucleic acid sequence: SEQ ID NO: 4)

### Example 2 Flow cytometry to evaluate antigen binding

Analysis of hchIgG1 ααCD37 b(chHH1) binding to CD37 expressing Daudi cells Cells stained and fixated.

The constructed chHH1 was analyzed for target binding by flow cytometry. Daudi cells express CD37, and the cells were stained with either chHH1 or hIgG1 αNIP. In the left panel the intact Daudi cells are selected, and in the right panel the fluorescence histogram of these cells stained are shown. chHH1 bound to the CD37-expressing Daudi cells (solid line) while the chimeric αNIP IgG₁ did not (dotted line).

### Example 3 Radiolabeling of chimeric HH1

Iodination: Antibodies were labeled with ¹²⁵I through indirect iodination using IODOGEN pre-coated iodination tubes (Pierce, Rockford, IL) according to the manufacturer's description.

Labeling with ¹¹¹In and ¹⁷⁷Lu: Antibodies were first reacted with a chelator (p-SCN-Bn-DTPA or p-SCN-Bn-DOTA).

The DTPA or DOTA chelator was dissolved in 0.05 M HCl, and then added to the antibody, which was pH-adjusted to approximately 8,5 by washing with carbonate buffer, in a 5:1 ratio. pH was then checked again and if necessary adjusted. The solution was shaken in 60 min at room temperature, and then the reaction was terminated by adding 50 µl 200 mM glycine solution (per mg antibody).

To remove free chelator the conjugated antibody was washed 4-5 times with PBS (PAA), and then adjusted to pH 5 by washing with ammonium acetate. ¹¹¹In or ¹⁷⁷Lu (Perkin Elmer, Boston, Ma, USA) was then added to 0.5 mg DOTA-Ab, and shaken for one hour at 42°C.

Finally, the product was purified by elution on a gel filtration column, e.g., Sephadex G-25 PD10 (GE health) or similar. The overall labeling yield varied from 17 % to 63 %.

The quality of the radioimmunoconjugates was measured using lymphoma cells and a modified Lindmo method (Example 4).

### Example 4 Immunoreactivity

Background: Immunoreactivity analysis was used to measure the antibody's ability to bind to antigen-positive target cells.

Methods: Two parallelsof 5, 10, 40, 100 og 300 million cells pr.ml in approximately 0.3 ml were used.

Half of the tubes were added unlabeled antibody to a concentration of 0.5 mg/ml and incubated for 15 min. to block the antigen.

Thereafter, 5-20 ng/ml radioimmunoconjugate was added to all tubes and the suspension incubated at 4°C using a shaker for approx. 2 hours.

Thereafter the tubes were centrifuged and the supernatant removed and stored for counting. The pellets were re-suspended in 1 ml PBS and centrifuged. This washing procedure was repeated twice and the supernatants pooled. Tubes containing the pellets or the pooled supernatants were counted on a gamma counter.

The specific bound activity was calculated using the following: Pellet counts nonblocked (PCN), combined supernatant counts (CSC) and the total applied (TA) for each sample. Specific binding (SB) was calculated as follows: (PCN/TA) - (PCN/TA)blocked = SB.

The SB values were calculated for each cell concentrations and plotted in a double reciprocal plot and the immunoreactive fraction at infinite antigen access was determined according Lindmo et al (J Immunol Methods. 1984 Aug 3;72(1):77-89).

Results: Measurements of two batches of ¹⁷⁷Lu-labeled chimeric HH1 gave immunoreactivities of 48.1% and 84.5% respectively. One batch of ¹²⁵I-labeled chimeric HH1 gave an immunoreactivity of 67.6%. In conclusion these data are in good agreement with those typically found for the murine HH1 and that show that the chimeric HH1 has a relevant antigen-targeting ability.

### Example 5 Binding to tumor cells in vitro

Introduction: A binding assay was performed in order to determine the equilibrium binding constand (K_{d}) of chHH1, the average number of antigens on Daudi cells (Bₘₐₓ) and and the assoiciation rate constant (ka) of chHH1 (Dahle et al., 2007).

Materials and methods: chHH1 was labeled with ¹²⁵I (example 3). 5 million cells per ml in 0.4 ml medium were suspended in tubes. One parallel of cells was blocked with 0.5 mg/ml of unlabeled chHH1 for 15 minutes before adding ¹²⁵I-labeled chHH1. One parallel of cells was not blocked. Both parallels were incubated with 100, 1000, 5000 and 10000 ng/ml of ¹²⁵I-HH1. The cells were incubated for 5, 10, 20, 30 minutes and 1, 1.5 and 2 hours. After incubation the cells were washed with PBS with 5 % foetal calf serum. The cells and the supernatant and washes were counted in a gamma counter. The experiment was repeated three times.

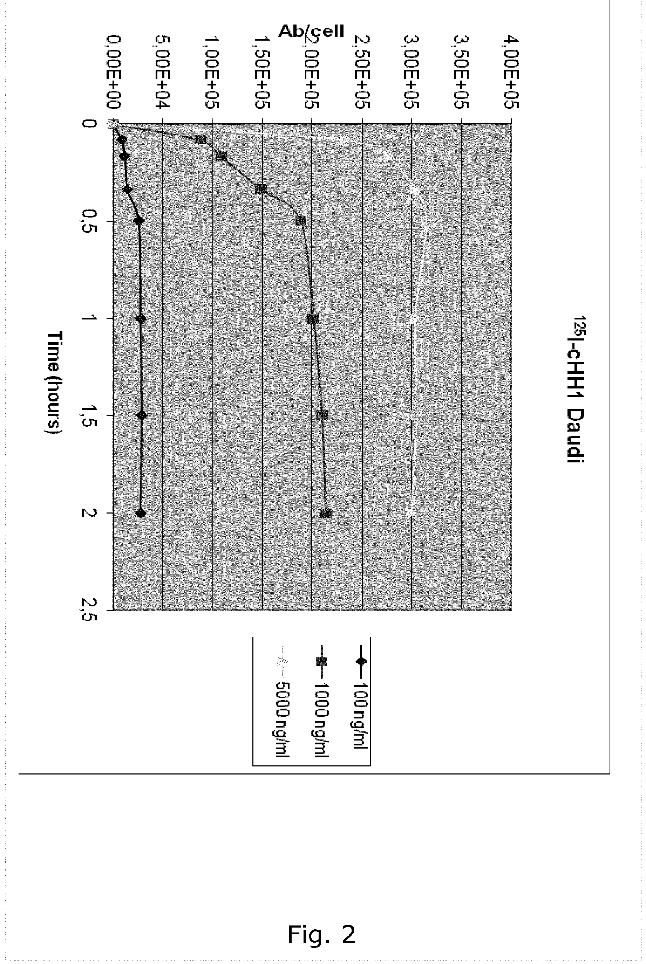

Results:Typical binding curves are shown in figure 2. The K_{d} of chHH1 was 3.0 ±0.3, the Bₘₐₓ of Daudi cells was 330000 ± 4000 CD37 antigens per cell and the kₐ of chHH1 was 0.36 ± 0.03 nM/h. The data was similar as for murine HH1 (K_{d} = 2.7 ±0.3, Bₘₐₓ = 340000 ± 5000 CD37 antigens per cell and kₐ 0.72 ± 0.03 nM/h).

### Example 6 Biodistribution and tumor targeting in mice

Biodistribution of ¹⁷⁷Lu-labeled chimeric HH1 was studied in male nude Balb/C mice.

Materials and methods: The radiolabeling was performed using p-SCN-Bn-DOTA as a bifunctional chelating agent to complex the radionuclide to the antibody (see Example 3). The preparation was administered by tail vein injection of 100 µl solution to each animal.

Male nude Balb/C mice with a body weight of 21-25 g were used.

An activity of 120 kBq was injected in each animal. Five animals were used per time point. Autopsies were performed after cervical dislocation at various time points after injection. The weight of each tissue sample was determined, and ¹¹¹In were measured by a calibrated gamma detector (Cobra II auto-gamma detector, Packard Instrument Company, Meriden, CT, USA). Samples of the injectates were used as references in the measurement procedures.

Results: The biodistribution of ¹⁷⁷Lu-chimeric HH1 at 1 hour, 20 hours and six days after injection is presented in Table 1. The data shows that the antibody has a relevant biodistribution similar to that observed with radiolabeled rituximab (Dahle et al, 2006 Nucl Med Biol, 33, 271-279). In conclusion, biodistribution of radiolabeled chimeric HH1 indicate that the antibody has relevant properties for in vivo use.

**Table 1 Biodistribution of ¹⁷⁷Lu-labeled chimeric HH1 in male nude Balb/C mice as percent of injected dose per gram ± SD.**

| Tissue | 1 h | 20 h | 6 days |
|---|---|---|---|
| Blood | 25.0 ± 2.9 | 15.2 ± 1.0 | 8.9 ± 3.5 |
| Lung | 7.0 ± 1.1 | 5.2 ± 0.9 | 2.9 ± 0.7 |
| Heart | 7.8 ± 0.8 | 5.8 ± 1.5 | 3.0 ± 1.0 |
| Liver | 10.8 ± 2.3 | 4.9 ± 0.1 | 3.9 ± 0.2 |
| Spleen | 9.6 ± 3.4 | 4.7 ± 0.2 | 4.8 ± 1.1 |
| Kidney | 8.0 ± 1.6 | 5.0 ± 0.4 | 3.3 ± 1.0 |
| Femur | 4.5 ± 2.4 | 2.8 ± 0.6 | 1.7 ± 0.3 |
| Stomach | 1.3 ± 0.5 | 1.0 ± 0.3 | 1.0 ± 0.5 |
| Small intestine | 3.1 ± 0.7 | 1.7 ± 0.1 | 1.3 ± 0.3 |
| Large intestine | 1.4 ± 0.3 | 1.4 ± 0.2 | 0.9 ± 0.2 |
| Brain | 0.7 ± 0.1 | 0.3 ± 0.3 | 0.3 ± 0.1 |

### Example 7 In vitro ADCC assay using flow cytometry viability measurements

Background: The ADCC property of chimeric HH1 antibody was evaluated using Natural killer (NK) cells and Daudi lymphoma cells.

Methods: Human blood was harvested from volunteers and treated with Lymphoprep and Dynabeads to obtained isolated NK cells. The Daudi cells were treated with DiOC18 to stain the cells.

Daudi target cells were labeled with 10µL of DiOC18 per 106 target cells/mL by incubating for for 30 minutes at 37oC. Cells were washed two times with PBS and re-suspend to 1 ml in cell culture medium.

The DiOC18 treated Daudi Cells were labeled with antibody by adding either chimeric HH1 or as a comparison rituximab to a concentration of 10 µg/ml and incubated for 15 minutes on ice before centrifugation and resuspension to remove unbound antibody.

Various amounts of NK cells were diluted to yield concentrations of 4x10⁵ /ml (40:1), 2x10⁵ /ml (20:1), 1x10⁵ /ml (10:1) and 5x10⁴ /ml (5:1) in cell culture medium. Propidium Iodide counterstain was prepared by adding 40 µL of PI/ml of culture medium to make counter-stain solution.

Labeled Daudi cells were dilluted to 10⁴/ml in culture medium. The assay was started by mixing 50 µL of effectors and 50 µL of Daudi cells into a reaction tube.

To the reaction tube was added 50µL of PI solution. After two hours of incubation at 37°C the percent of surviving Daudi cells were evaluated using flow cytometry.

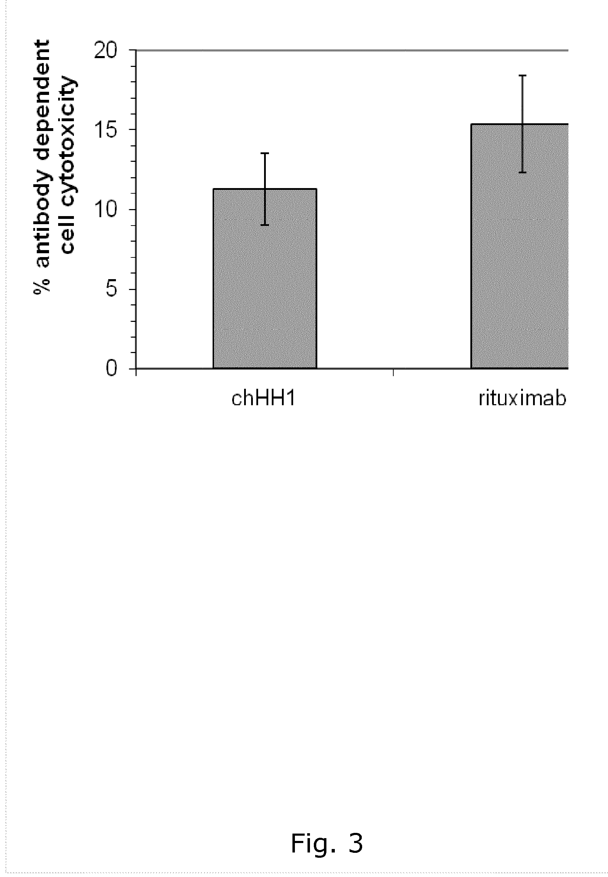

For chHH1 11 % of the Daudi cells died by ADCC, while for rituximab 15 % of the cells died by ADCC. The percentage induction of ADCC was not significantly different for the two antibodies (see figure 3).

### Example 8 In vitro ADCC cell assay using interleukin stimulated PBMC

Background: ADCC activity of murine and chimeric versions of mAb HH1 was evaluated by by Cr51 release assay.

Materials and Methods: The ability of murine HH1 and chimeric HH1 to mediate antibody-dependent cell-mediated cytotoxicity (ADCC) was assessed using Daudi cells as target cells and IL2-stimulated human PBMCs as effector cells.

Daudi cells (Human Burkitt's lymphoma cell line) were grown in tissue culture flasks (175 cm 2) using RPMI-1640, supplemented with 10% heat-inactivated fetal bovine serum, 1 mM sodium pyruvat, 2 mM L-glutamine and 1% Pen/Strep. The cultures were maintained at a cell concentration between 3×10⁵ and 1.5×10⁶/ml by sub-cultivation with fresh culture medium 2-3 times a week. An aliquot of the cell culture at a cell density between 0.5×10⁶/ml and 1.0 ×10⁶/ml is centrifuged (1200 rpm) for 5 min.

Cells were washed once with washing buffer (DPBS with 2 % FCS) and pelleted (1200 rpm; 5 min). Cell pellet was resuspended in assay medium [RPMI w/HEPES, 10% FCS] and cell count was determined. Cell concentration was adjusted to 1×10⁶/ml for labeling with chromium-51.

Approximately 30-50 ml whole blood drawn on EDTA glass from healthy donors was used for the isolation of PBMC. Whole blood was diluted 1:1 with DPBS (Hanks' Balanced Salt Solution w/o calcium and magnesium) in a 50 ml tube.

Diluted whole blood was layered on top of Lymphoprep (Medinor) in a ratio 2:1 in a 50 ml tube and centrifuged at 1000xg for 20 min with slow braking. The mononuclear cells from the interface were aspirated and washed twice with DPBS (300xg, 10 min).

The pelleted cells were gently resuspended in culture medium (RPMI-1640 with 10% heat-inactivated fetal bovine serum, 1% Pen/Strep) using a pipette and the cell count was determined in the cell counter.

The PBMC concentration was adjusted to 5×10⁵/ml and maintained in culture medium supplemented with 25ng/ml IL-2 (eBiosciences) and were cultured in 6 wells culture dish at 37° C in CO 2 incubator for 3 days. IL-2 stimulated PBMC were collected, counted and resuspended in assay medium [RPMI w/HEPES, 10% FCS] at a concentration of 1.5×10⁶/ml.

1×10⁶ Daudi cells were labeled with 3,7 MBq chromium-51 (Cr51) in a volume of 1 ml at 37° C for 1 hour.

The labeled cells were washed in DPBS with 2 % FCS three times by centrifugation (2000 rpm at 5 min).

The labeled cells were aliquoted in equal volumes for binding of murine and chimeric HH1 including a control with no antibody present. All samples were incubated at 37° C for 10 min, and then washed twice in DPBS with 2 % FCS three times by centrifugation (2000 rpm at 5 min).

The co-cultivation of effector cells with target cells was performed in triplicates in 96-well round-bottom microtiter plates in a final volume of 200 µl.

First 10.000 target cells in a volume of 100 µl in assay medium were plated, followed by effector cells in a volume of 100 µl in at different concentrations to achieve the effector: target ratios assayed.

As a control, target cells were cultivated either in assay medium alone (spontaneous lysis) or in assay medium supplemented with 1% Triton X-100 (maximal lysis).

The co-culture was incubated at 37° C. in a humid CO 2 incubator for 4 hours. The cytotoxic effect was measured by Cr51 release into the supernatant. At the end of the incubation cells were removed from the culture medium by centrifugation (1500 rpm; 5 min) at room temperature. Cell free supernatants (100 µl/well) were transferred into 96 corresponding 0.2 ml micro tubes. The amount of Cr51 release was measured by counting in a Packard Cobra II Gamma counter.

Results: The results of the experiment are presented in Table 2. As shown the cell lysis of Daudi cells pretreated with the chimeric HH1 was significant for all three blood donors.

Pretreatment with the murine HH1 did not seem to cause any significant lysis compared to Daudi cells not pretreated with antibody.

**Table 2. In vitro cell lysis after treatment with interleukin stimulated PBMC effector cells versus Daudi lymphoma target cells with pretreatment with anti-CD37 antibody**

| Effector to target ratio | Chimeric HH1 (% lysis) | | Murine HH1 (% lysis) | |
|---|---|---|---|---|
| | Mean | Range | Mean | Range |
| 40:1 | 159.2% | 122.6-208.9% | 106.5% | 99.4-111.8% |
| 10:1 | 177.9% | 133.8-235.5% | 105.5% | 100.7-111.3% |

Effector cells from three individuals. Data were normalized to the measured cell lysis without antibody which was chosen as 100%.

Conclusion: When assayed using human PBMC in vitro, chimeric HH1 causes a significant ADCC in terms of cell lysis on lymphoma cells in vitro while murine HH1 did not affect any significant cell lysis effect compared to no treatment.

### Example 9 Binding comparison between chimeric and murine HH1

To evaluate whether the chimeric HH1 had retained the antigen and epitope binding capability cells were saturated with chimeric and murine HH1 and then the ability to block the binding of radiolabeled HH1 was assessed.

Materials and methods: A cell suspension of Daudi cells was distributed among 6 tubes with 1.7 million cells in 0.2 ml PBS in each tube. Tubes 1 and 2 were kept unblocked, tubes 3 and 4 were blocked by adding 3 µg of HH1 in 5 µl and tubes 5 and 6 were added 3 µg of chimeric HH1 in 5 µl.

The tubes were incubated for 10 minutes and thereafter added 2 ng of 1251 labeled HH1 and further incubated for 40 minutes at room temperature using a cell shaker.

The total activity in each tube was measured using a LKB gamma counter and thereafter the cells were washed three times with 1 ml PBS with 0.5% BSA and 1 mM DTPA. The final pellets were counted for radioactivity to determine the cell bound activity.

Results: It was found that the non-blocked cells retained 42.7% of the activity (range 42.7%-42.7%). Cells Blocked with HH1 retained 1.6% of the activity (range 1.4%-1.8%). Cells blocked with chimeric HH1 retained 1.3% of the activity (range 1.1%-1.5%).

In conclusion, the binding of radiolabeled murine HH1 is blocked highly effective with chimeric HH1 and equal to that of blocking with murine HH1 indicating that the antigen and epitope binding ability is maintained for the chimeric version of HH1.

This also indicate that the chimeric HH1 could be useful as pretreatment for blocking CD37 antigen in normal tissues prior to therapy with radioimmunoconjugate or immuntoxin versions of HH1.

### Example 10: ADCC for chHH1.1 with Rec-1 cells as target cells

### Materials and methods:

Blood was drawn from three healthy individuals into EDTA glasses and used for peripheral blood mononuclear cells (PBMC) isolation. Whole blood was diluted 1:1 with DPBS (DPBS, Hyclone, Thermo Scientific, USA) in a 50 ml tube. Diluted whole blood was layered on top of Lymphoprep (Medinor, Norway) in a ratio 2:1 in a 50 ml tube and centrifuged at 1000×g for 20 min with slow braking.

The mononuclear cells from the interface were aspirated and washed twice with DPBS (300xg, 10 min). The pelleted cells were gently resuspended in culture medium (RPMI-1640 with 10% heat-inactivated fetal bovine serum) using a pipette and the cell count was determined in the cell counter.

PBMC were stimulated with 25 ng/ml human recombinant IL-2 (eBiosciences) for 3 days.
Rec-1 mantle cell lymphoma cells (LG Standards, Boras, Sweden) were used as target cells in the cytotoxity assay. They were grown in tissue culture flasks (175 cm²) using RPMI-1640 (Hyclone, Thermo Scientific, USA), supplemented with 10 % heat-inactivated fetal bovine serum (PAA, Thermo Scientific, USA).

Cell pellet was resuspended in assay medium (RPMI, 10% FCS) and cell count determined. Cell concentration was adjusted to 5×10⁶ /ml for labeling with chromium-51.

5×10⁶ cells were labeled with 3,7 MBq chromium-51 (⁵¹Cr) (PerkinElmer, Netherlands) in a volume of 1 ml at 37° C for 1 hour. The labeled cells were washed three times in DPBS with 2 % FCS three times by centrifugation (2000 rpm at 5 min).

The labeled cells were aliquoted in equal volumes for binding of 20 µg/ml murine HH1, 20 µg/ml rituximab, 20 µg/ml chHH1.1 (IgG1 isotype) or a combination of 20 µg/ml rituximab and 20 µg/ml chHH1.1 including a control with no antibody present. All samples were incubated at 37° C for 10 min, and then washed twice in DPBS with 2 % FCS three times by centrifugation (2000 rpm at 5 min).

The effector cells were cultivated with target cells in a ratio of 40:1, 20:1 or 10:1 in 96-well round-bottom microtiter plates in a final volume of 200 µl. First 10.000 target cells in a volume of 100 µl in assay medium are plated, followed by human PBMC in a volume of 100 µl. As controls, target cells were cultivated in assay medium alone (spontaneous lysis) and in assay medium supplemented with 1% Triton X-100 (maximal lysis; total release).

All samples were run in triplicates. The co-culture was incubated at 37° C in a humid CO 2 incubator for 4 hours. The cytotoxic effect was measured by ⁵¹Cr release into the supernatant. At the end of the incubation cells were removed from the culture medium by centrifugation (1500 rpm; 5 min) at room temperature. Cell free supernatants (150 µl/well) were transferred into 96 corresponding 0.2 ml micro tubes. The amount of 51Cr released was measured by a gamma counter (Cobra II, Packard, land).

% specific lysis was calculated with the following equation: 100 x (experimental release - spontaneous release) /(total release - spontaneous release). Experimental release being the mean value of the replicates of a sample treatment. Triplicate samples for spontane release and total release were run for each antibody treatment group, and the mean values were used for the experimental samples of the respective antibody treatment.

### Results:

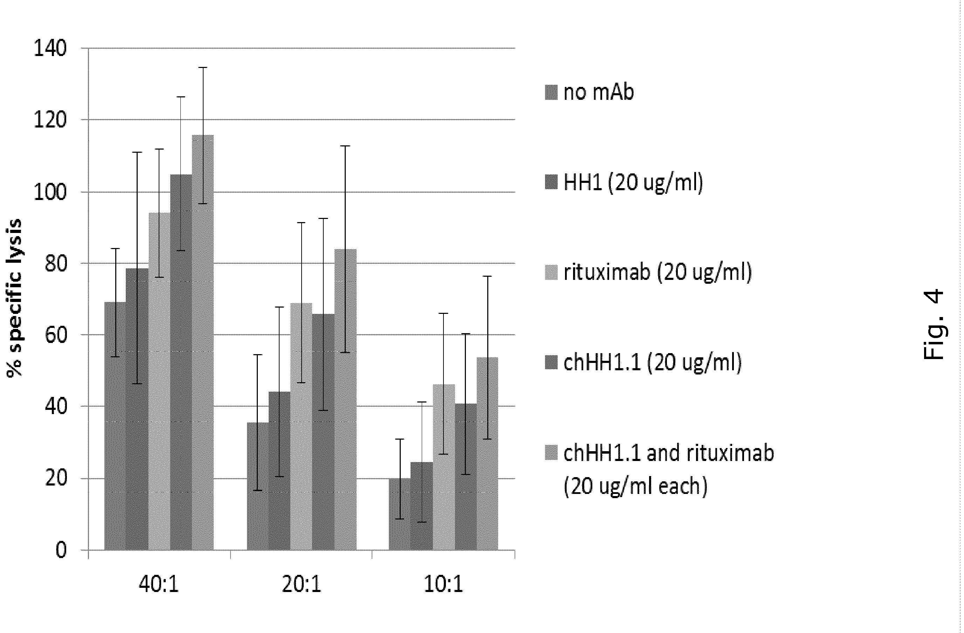

Figure 4 show that chHH1.1 was equally good as rituximab and slightly better than the murine HH1 in inducing specific lysis by ADCC in Rec-1 cells. The combination of chHH1 and rituximab gave a slightly higher specific lysis than treatment with the two antibodies alone.

### Conclusion:

ADCC is an active mechanism of action for chHH1.1 in Rec-1 cells. Combination treatment with rituximab and chHH1.1 gave slightly higher ADCC than either antibody alone.

### Example 11: CDC for chHH1.1 with Rec-1 cells as target cells

### Materials and methods:

Blood was drawn from three healthy individuals and serum was isolated by centrifugation.

Rec-1 mantle cell lymphoma cells (LG Standards, Boras, Sweden) were used as target cells in the cytotoxity assay. They were grown in tissue culture flasks (175 cm²) using RPMI-1640 (Hyclone, Thermo Scientific, USA), supplemented with 10 % heat-inactivated fetal bovine serum (PAA, Thermo Scientific, USA).

Cell pellet was resuspended in assay medium (RPMI, 10% FCS) and cell count determined. Cell concentration was adjusted to 5×10⁶/ml for labeling with chromium-51.

5×10⁶ cells were labeled with 3, 7 MBq chromium-51 (⁵¹Cr) (PerkinElmer, Netherlands) in a volume of 1 ml at 37° C for 1 hour. The labeled cells were washed three times in DPBS with 2 % FCS three times by centrifugation (2000 rpm at 5 min). The labeled cells were aliquoted in equal volumes for binding of 20 µg/ml murine HH1, 20 µg/ml rituximab, 20 µg/ml chHH1.1 (IgG1 isotype) or a combination of 20 µg/ml rituximab and 20 µg/ml chHH1.1 including a control with no antibody present. All samples were incubated at 37° C for 10 min, and then washed twice in DPBS with 2 % FCS three times by centrifugation (2000 rpm at 5 min).

The target cells were cultivated with 10 % serum in 96-well round-bottom microtiter plates in a final volume of 200 µl. As controls, target cells were cultivated in assay medium alone (spontaneous lysis) and in assay medium supplemented with 1% Triton X-100 (maximal lysis; total release).

All samples were run in triplicates. The co-culture was incubated at 37° C in a humid CO 2 incubator for 2 hours. The cytotoxic effect was measured by 51Cr release into the supernatant. At the end of the incubation cells were removed from the culture medium by centrifugation (1500 rpm; 5 min) at room temperature. Cell free supernatants (150 µl/well) were transferred into 96 corresponding 0.2 ml micro tubes. The amount of ⁵¹Cr released was measured by a gamma counter (Cobra II, Packard, land).

% specific lysis was calculated with the following equation: 100 x (experimental release - spontaneous release)/(totalt release - spontaneous release). Experimental release being the mean value of the replicates of a sample treatment. Triplicate samples for spontane release and total release were run for each antibody treatment group, and the mean values were used for the experimental samples of the respective antibody treatment.

### Results:

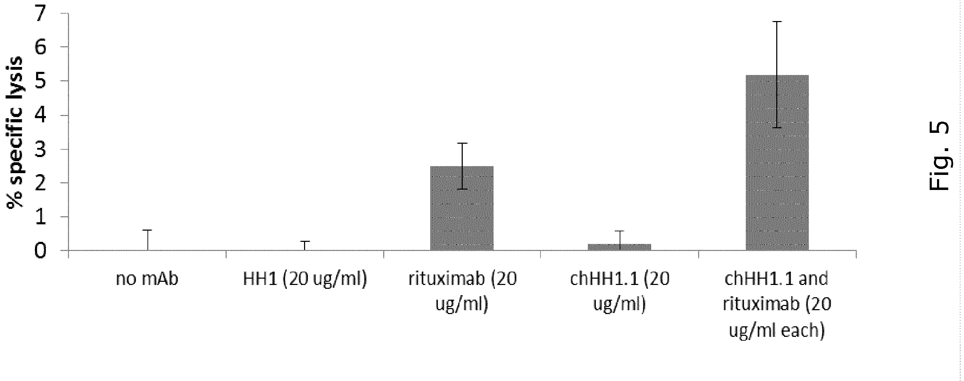

Figure 5 shows that chHH1.1 do not work via a CDC mechanism alone, but chHH1.1 and rituximab together had a larger effect on specific lysis than the added effect of rituximab and chHH1.1 alone.

### Conclusion:

CDC is not an active mechanism of action for chHH1.1 alone. There was, however, a synergistic effect of co-treatment with chHH1.1 and rituximab.

### Example 12: Binding of ¹²⁵I-chHH1.3 to Ramos cells

### Materials and methods:

Chimeric HH1 with isotype IgG3 (chHH1.3) and murine HH1 (mHH1) were radiolabelled with ¹²⁵I using indirect iodination method according to method supplied by manufacturer of iodination tubes (Pierce, UK). In one experiment Ramos cells were blocked with 20 mg chHH1.3 or mHH1 and radiolabelled with ¹²⁵I-chHH1.3 in order to measure non-specific binding.

In another experiment Ramos cells were blocked with 20 mg ch HH1.3, mHH1 or chHH1.1 and subsequently radiolabelled with ¹²⁵I-mHH1 to measure non-specific binding. Binding to unblocked cells were used in both experiments to measure total binding.

DNA chHH1.3 Fc sequence with mutation full length heavy chain can be seen as SEQ ID NO: 10.

Amino acid chHH1.3 Fc sequence with mutation full length light chain can be seen as SEQ ID NO: 11.

DNA chHH1.3 Fc sequence with mutation full length heavy chain can be seen as SEQ ID NO: 12.

Amino acid chHH1.3 Fc sequence with mutation full length light chain can be seen as SEQ ID NO:13.

chHH1.3 Fc sequence without mutation (constant region) can be seen as SEQ ID NO: 14.

### Results:

When unblocked Ramos cells were radiolabeld with ¹²⁵I-chHH1.3 the total binding was 81 % and when Ramos cells were radiolabeled with ¹²⁵I-mHH1 the total binding was 77 %. However, when cells blocked with chHH1.3 or mHH1 were radiolabeled with ¹²⁵I-chHH1.3 the non-specific binding was 0.8 % and 44 % respectively, indicating that the chHH1.3 has higher affinity than mHH1 for the CD37 antigen. Furthermore, when cells blocked with chHH1.3, mHH1 or chHH1.1 were radiolabeled with ¹²⁵I-mHH1 the non-specifc binding was 0.3 %, 0.5 % and 0.7 %, respectively, indicating that the three antibodies bind to the same epitope on the CD37 antigen.

**Table 3. Binding of ¹²⁵I-chHH1.3 and 125I-mHH1 to Ramos cells.**

| RIC | Non-blocked | Blocked with 20 µg/ml cold antibody | | |
|---|---|---|---|---|
| | | mHH1 | chHH1.1 | chHH1.3 |
| ¹²⁵I-chHH1.3 | 81 % | 44 % | Not done | 0.8 % |
| ¹²⁵I-mHH1 | 77 % | 0.5 % | 0.7 % | 0.3 % |

### Conclusion:

The results indicated that mHH1, chHH1.1 and chHH1.3 bind to the same epitope of CD37. Unexpectedly, the affinity of chHH1.3 was higher than for mHH1.

### Example 13: Biodistribution of ¹²⁵I-chHH1.3 in nude mice

### Materials and methods:

Chimeric HH1 with isotype IgG3 (chHH1.3) was radiolabelled with ¹²⁵I using indirect iodination method according to method supplied by manufacturer of iodination tubes (Pierce, UK).

The preparations were administered by tail vein injection of 100 µl solution to each animal. An activity of 600 kBq was injected per mice. Two animals were used per time point. Autopsies were performed after cervical dislocation 24 hours and 48 hours after injection. The weight of each tissue sample was determined, and the activity of ¹²⁵I in each organ sample was measured by a calibrated gamma detector (Cobra II auto-gamma detector, Packard Instrument Company, Meriden, CT, USA). Samples of the injectates were used as references in the measurement procedures. The decay corrected percentages of the injected dose per gram tissue (%ID/g) was calculated for each time point.

### Results and conclusion:

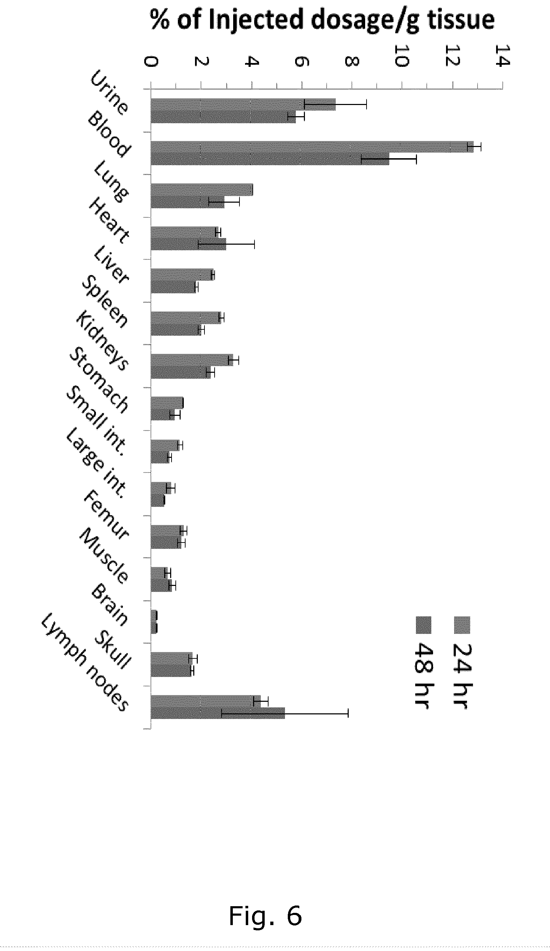

The chHH1.3 antibody showed a relevant distribution in nude mice (Figure 6).

Figure 6. Biodistribution (% I.D./g) of ¹²⁵I-chHH1.3 in female nude mice 24 and 48 hours after injection.

### Example 14: ADCC and CDC activity of chHH1.3 on Daudi cells

### Materials and methods:

ADCC and CDC experiments were performed using the same procedures as described in Example 10 and 11, except that a commercially available serum was used for the CDC assay and that the PBMC were stimulated over night with 25 ng/ml human recombinant IL-2 (eBiosciences) before isolation of natural killer cells using the same assay as described in Example 7. For isolation of PBMC, blood was drawn from two individuals into EDTA tubes.

Daudi lymphoma cells (LG standards, Boras, Sweden) (target cells) were used as target cells in the cytotoxity assay. They were grown in tissue culture flasks (175 cm²) using RPMI-1640 (Hyclone, Thermo Scientific, USA), supplemented with 10 % heat-inactivated fetal bovine serum (PAA, Thermo Scientific, USA).

Cell pellet was resuspended in assay medium (RPMI, 10% FCS) and cell count determined. Cell concentration was adjusted to 5×10⁶/ml for labeling with chromium-51.

5×10⁶ Daudi cells were labeled with 4 MBq chromium-51 (⁵¹Cr) (PerkinElmer, Netherlands) in a volume of 1 ml at 37° C for 1 hour. The labeled cells were washed three times in DPBS with 2 % FCS three times by centrifugation (2000 rpm at 5 min). The labeled cells were aliquoted in equal volumes for binding of 20 mg/ml chHH1.3 antibody or a combination of chHH1.3 and rituximab, both 20 mg/ml, and a control without antibody. All samples were incubated at 37° C for 10 min, and then washed twice in DPBS with 2 % FCS three times by centrifugation (2000 rpm at 5 min).

For the CDC assay the Daudi target cells were cultivated with 10 and 30 % serum in 96-well round-bottom microtiter plates in a final volume of 200 µl. For ADCC the NK effector cells were cultivated with Daudi target cells in a ratio of 10:1 (individual 1) or 15:1 (individual 2) in 96-well round-bottom microtiter plates in a final volume of 200 µl.

As controls, target cells were cultivated in assay medium alone (spontaneous lysis) and in assay medium supplemented with 1% Triton X-100 (maximal lysis; total release).

All samples were run in triplicates. The co-culture was incubated at 37° C in a humid CO₂ incubator for 2 hours. The cytotoxic effect was measured by ⁵¹Cr release into the supernatant. At the end of the incubation cells were removed from the culture medium by centrifugation (1500 rpm; 5 min) at room temperature. Cell free supernatants (150 µl/well) were transferred into 96 corresponding 0.2 ml micro tubes. The amount of ⁵¹Cr released was measured by a gamma counter (Cobra II, Packard, land).

% specific lysis was calculated with the following equation: 100 x (experimental release - spontaneous release)/(totalt release - spontaneous release). Experimental release being the mean value of the replicates of a sample treatment. Triplicate samples for spontane release and total release were run for each antibody treatment group, and the mean values were used for the experimental samples of the respective antibody treatment.

### Results:

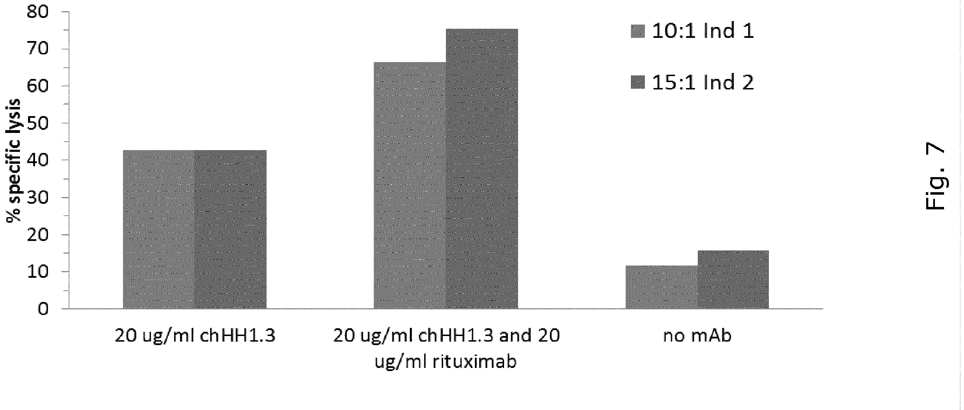

The chHH1.3 antibody did not induce specific lysis in Daudi B-lymphoma cells by the CDC mechanism. There was, however, a clear effect of the chHH1.3 antibody on specific lysis in Daudi cells by the ADCC mechanism (Figure 7).

### Conclusion:

CDC is not an active mechanism while ADCC is an active mechanism for chHH1.3 with Daudi cells as target.

## Claims

1. A radioimmunoconjugate that binds human CD37 comprising:
a) an antibody molecule that binds to human CD37, and that is a chimeric antibody, defined by:
i) a variable heavy chain comprising an amino acid sequence encoded by the nucleic acid sequence shown in SEQ ID NO: 1;
ii) a variable light chain comprising an amino acid sequence encoded by the nucleic acid sequence shown in SEQ ID NO:3,
iii) constant heavy and light chains that are of human origin, wherein said constant heavy chain i) comprises amino acid sequences encoded by the nucleic acid sequences shown in SEQ ID NO:5, SEQ ID NO:6, and SEQ ID NO:7 and wherein said constant light chain ii) comprises an amino acid sequence encoded by the nucleic acid sequence shown in SEQ ID NO:9,
b) a linker, and
c) a ²¹²Pb radionuclide.

2. The radioimmunoconjugate according to claim 1, wherein the linker is selected from the group consisting of p-SCN-bn-DOTA, DOTA-NHS-ester, p-SCN-Bn-TCMC, p-SCN-Bn-DTPA and CHX-A"-DTPA.

3. A pharmaceutical composition comprising a radioimmunoconjugate according to claims 1-2, and a pharmaceutically acceptable carrier and/or excipient.

4. A pharmaceutical composition according to claim 3, for use in treating B-cell malignancies.

5. The pharmaceutical composition for use according to claim 4, wherein the B-cell malignancy is selected from the group consisting of B-cell non-Hodgkin's lymphoma, B-cell chronic lymphocytic leukemia, hairy cell leukemia, lymphoplasmacytic lymphoma and multiple myeloma.

6. The pharmaceutical composition for use according to claims 4 and 5 wherein the radioimmunoconjugate is administered in combination with or in addition to other therapy.

7. The pharmaceutical composition for use according to claim 6, wherein the other therapy is selected from the group consisting of pretreatment, chemotherapy, monoclonal antibody therapy, surgery, radiotherapy, and photodynamic therapy.

8. A kit for the production of the radioimmunoconjugate according to of claims 1-2, comprising two or more vials, wherein one vial contains a conjugate comprising a chelator linked to an antibody according to claims 1-2; and a second vial contains a ²¹²Pb radionuclide.

9. An injectable preparation of chimeric antibody as defined in claim 1, for use in pretreatment of a B-cell malignancy, wherein CD37 is blocked in normal tissues before radioimmunotherapy with radiolabeled or immunotoxic versions of monoclonal antibody HH1.

10. The preparation for use according to claim 9, wherein the amount of chimeric antibody is at least 0.5 mg and not more than 1 g of antibody.

## Patentansprüche

1. Radioimmunkonjugat, das menschliches CD37 bindet, umfassend:
a) ein Antikörpermolekül, das an menschliches CD37 bindet, und das ein chimärer Antikörper ist, definiert durch:
i) eine variable schwere Kette, die eine Aminosäuresequenz umfasst, welche durch die in SEQ ID NO:1 gezeigte Nukleinsäuresequenz kodiert wird;
ii) eine variable leichte Kette, die eine Aminosäuresequenz umfasst, welche durch die in SEQ ID NO:3 gezeigte Nukleinsäuresequenz kodiert wird,
iii) konstante schwere und leichte Ketten, die menschlichen Ursprungs sind, wobei die konstante schwere Kette i) Aminosäuresequenzen umfasst, die durch die in SEQ ID NO:5, SEQ ID NO:6 und SEQ ID NO:7 gezeigten Nukleinsäuresequenzen kodiert werden, und wobei die konstante leichte Kette ii) eine Aminosäuresequenz umfasst, die durch die in SEQ ID NO:9 gezeigte Nukleinsäuresequenz codiert wird,
b) einen Linker und
c) ein ²¹²Pb-Radionuklid.

2. Radioimmunkonjugat nach Anspruch 1, wobei der Linker ausgewählt ist aus der Gruppe bestehend aus p-SCN-bn-DOTA, DOTA-NHS-Ester, p-SCN-Bn-TCMC, p-SCN-Bn-DTPA und CHX-A''-DTPA.

3. Pharmazeutische Zusammensetzung, umfassend ein Radioimmunkonjugat nach Anspruch 1-2 und einen pharmazeutisch annehmbaren Träger und/oder Hilfsstoff.

4. Pharmazeutische Zusammensetzung nach Anspruch 3 zur Verwendung bei der Behandlung von B-Zell-Malignomen.

5. Pharmazeutische Zusammensetzung zur Verwendung nach Anspruch 4, wobei das B-Zell-Malignom ausgewählt ist aus der Gruppe bestehend aus B-Zell-Non-Hodgkin-Lymphom, chronischer lymphatischer B-Zell-Leukämie, Haarzell-Leukämie, lymphoplasmacytischem Lymphom und multiplem Myelom.

6. Pharmazeutische Zusammensetzung zur Verwendung nach Anspruch 4-5, wobei das Radioimmunkonjugat in Kombination mit oder zusätzlich zu einer anderen Therapie verabreicht wird.

7. Pharmazeutische Zusammensetzung zur Verwendung nach Anspruch 6, wobei die andere Therapie ausgewählt ist aus der Gruppe bestehend aus Vorbehandlung, Chemotherapie, monoklonaler Antikörpertherapie, Operation, Strahlentherapie und photodynamischer Therapie.

8. Kit zur Herstellung des Radioimmunkonjugats nach Anspruch 1-2, umfassend zwei oder mehr Ampullen, wobei eine Ampulle ein Konjugat enthält, das einen Chelator umfasst, der an einen Antikörper nach Anspruch 1-2 gebunden ist; und eine zweite Ampulle ein ²¹²Pb-Radionuklid enthält.

9. Injizierbares Präparat eines chimären Antikörpers nach Anspruch 1 zur Verwendung bei der Vorbehandlung eines B-Zell-Malignoms, wobei CD37 in normalen Geweben vor der Radioimmuntherapie mit radioaktiv markierten oder immuntoxischen Versionen von HH1 blockiert wird.

10. Präparat zur Verwendung nach Anspruch 9, wobei die Menge an chimärem Antikörper mindestens 0,5 mg und nicht mehr als 1 g Antikörper beträgt.

## Revendications

1. Radioimmunoconjugué qui se lie au CD37 humain comprenant :
a) une molécule d'anticorps qui se lie au CD37 humain, et qui est un anticorps chimérique, défini par :
i) une chaîne lourde variable comprenant une séquence d'acides aminés codée par la séquence d'acide nucléique représentée dans la SEQ ID NO: 1 ;
ii) une chaîne légère variable comprenant une séquence d'acides aminés codée par la séquence d'acide nucléique représentée dans la SEQ ID NO: 3 ,
iii) des chaînes lourde et légère constantes qui sont d'origine humaine, dans lequel ladite chaîne lourde constante i) comprend des séquences d'acides aminés codées par les séquences d'acide nucléique représentées dans la SEQ ID NO: 5, la SEQ ID NO: 6, et la SEQ ID NO: 7 et dans lequel ladite chaîne légère constante ii) comprend une séquence d'acides aminés codée par la séquence d'acide nucléique représentée dans la SEQ ID NO: 9,
b) un lieur, et
c) un radionucléide de ²¹²Pb.

2. Radioimmunoconjugué selon la revendication 1, dans lequel le lieur est choisi dans le groupe constitué de p-SCN-bn-DOTA, DOTA-NHS-ester, p-SCN-Bn-TCMC, p-SCN-Bn-DTPA et CHX-A"-DTPA.

3. Composition pharmaceutique comprenant un radioimmunoconjugué selon les revendications 1 et 2 et un support pharmaceutiquement acceptable et/ou un excipient.

4. Composition pharmaceutique selon la revendication 3, destinée à être utilisée dans le traitement des tumeurs malignes à cellules B.

5. Composition pharmaceutique destinée à être utilisée selon la revendication 4, dans laquelle la tumeur maligne à cellules B est choisie dans le groupe constitué par le lymphome non hodgkinien à cellules B, la leucémie lymphoïde chronique à cellules B, la leucémie à tricholeucocytes, le lymphome lymphoplasmocytaire et le myélome multiple.

6. Composition pharmaceutique destinée à être utilisée selon les revendications 4 à 5, dans laquelle le radioimmunoconjugué est administré en combinaison avec ou en plus d'une autre thérapie.

7. Composition pharmaceutique destinée à être utilisée selon la revendication 6, dans laquelle l'autre thérapie est choisie dans le groupe comprenant le prétraitement, la chimiothérapie, la thérapie par anticorps monoclonaux, la chirurgie, la radiothérapie et la thérapie photodynamique.

8. Kit pour la production du radioimmunoconjugué selon les revendications 1 et 2, comprenant deux flacons ou plus, dans lequel un flacon contient un conjugué comprenant un chélateur lié à un anticorps selon les revendications 1 et 2 ; et un second flacon contient un radionucléide de ²¹²Pb.

9. Préparation injectable d'anticorps chimérique selon la revendication 1, destinée à être utilisée dans le prétraitement d'une tumeur maligne à cellules B, dans laquelle le CD37 est bloqué dans les tissus normaux avant la radioimmunothérapie avec des versions radiomarquées ou immunotoxiques de HH1.

10. Préparation destinée à être utilisée selon la revendication 9, dans laquelle la quantité d'anticorps chimérique est d'au moins 0,5 mg et pas plus de 1 g d'anticorps.


### SEQUENCE LISTING

<110> Nordic Nanovector AS Larsen, Roy H Dahle, Jostein
<120> Chimeric therapeutic antibodies
<130> 49202PC01
<150> US 61/569,981
   <151> 2011-12-13
<160> 14
<170> PatentIn version 3.5
<210> 1
   <211> 119
   <212> PRT
   <213> Mus musculus
<400> 1
   Glu Ile Gln Leu Gln Gln Ser Gly Pro Glu Leu Val Lys Pro Gly Ala
   1               5                   10                  15
   Ser Val Lys Val Ser Cys Lys Ala Ser Gly Tyr Ser Phe Thr Asp Tyr
               20                  25                  30
   Asn Met Tyr Trp Val Lys Gln Ser His Gly Lys Ser Leu Glu Trp Ile
           35                  40                  45
   Gly Tyr Ile Asp Pro Tyr Asn Gly Asp Thr Thr Tyr Asn Gln Lys Phe
       50                  55                  60
   Lys Gly Lys Ala Thr Leu Thr Val Asp Lys Ser Ser Ser Thr Ala Phe
   65                  70                  75                  80
   Ile His Leu Asn Ser Leu Thr Ser Glu Asp Ser Ala Val Tyr Tyr Cys
                   85                  90                  95
   Ala Arg Ser Pro Tyr Gly His Tyr Ala Met Asp Tyr Trp Gly Gln Gly
               100                 105                 110
   Thr Ser Val Thr Val Ser Ser
           115
<210> 2
   <211> 357
   <212> DNA
   <213> Mus musculus
<400> 2
   gagatccagc tgcagcagtc tggacctgag ctggtgaagc ctggggcttc agtgaaggta      60
   tcctgcaagg cttctggtta ctcattcact gactacaaca tgtactgggt gaagcagagc     120
   catggaaaga gccttgagtg gattggatat attgatcctt acaatggtga tactacctac     180
   aaccagaagt tcaagggcaa ggccacattg actgttgaca agtcctccag cacagccttc     240
   atccatctca acagcctgac atctgaggac tctgcagtct attactgtgc aagatcccct     300
   tatggtcact atgctatgga ctactggggt caaggaacct cagtcaccgt ctcctca        357
<210> 3
   <211> 107
   <212> **PRT**
   <213> Mus musculus
<400> 3
   Asp Ile Val Met Thr Gln Ser His Lys Leu Leu Ser Thr Ser Val Gly
   1               5                   10                  15
   Asp Arg Val Ser Ile Thr Cys Lys Ala Ser Gln Asp Val Ser Thr Ala
               20                  25                  30
   Val Asp Trp Tyr Gln Gln Lys Pro Gly Gln Ser Pro Lys Leu Leu Ile
           35                  40                  45
   Asn Trp Ala Ser Thr Arg His Thr Gly Val Pro Asp Arg Phe Thr Gly
       50                  55                  60
   Ser Gly Ser Gly Thr Asp Tyr Thr Leu Thr Ile Ser Ser Met Gln Ala
   65                  70                  75                  80
   Glu Asp Leu Ala Leu Tyr Tyr Cys Arg Gln His Tyr Ser Thr Pro Phe
                   85                  90                  95
   Thr Phe Gly Ser Gly Thr Lys Leu Glu Ile Lys
               100                 105
<210> 4
   <211> 321
   <212> DNA
   <213> Mus musculus
<400> 4
   gacattgtga tgacccagtc tcacaaactc ttgtccacat cagtaggaga cagggtcagc        60
   atcacctgca aggccagtca ggatgtgagt actgctgtag actggtatca acagaaacca       120
   ggacaatctc ctaaactact gattaactgg gcatccaccc ggcacactgg agtccctgat       180
   cgcttcacag gcagtggatc tgggacagat tatactctca ccatcagcag tatgcaggct       240
   gaagacctgg cactttatta ctgtcgacaa cattatagca ctccattcac gttcggctcg       300
   gggacaaagt tggaaataaa a                                                 321
<210> 5
   <211> 294
   <212> DNA
   <213> Homo sapiens
<400> 5
   cctccaccaa gggcccatcg gtcttccccc tggcaccctc ctccaagagc acctctgggg        60
   gcacagcggc cctgggctgc ctggtcaagg actacttccc cgaaccggtg acggtgtcgt       120
   ggaactcagg cgccctgacc agcggcgtgc acaccttccc ggctgtccta cagtcctcag       180
   gactctactc cctcagcagc gtggtgaccg tgccctccag cagcttgggc acccagacct       240
   acatctgcaa cgtgaatcac aagcccagca acaccaaggt ggacaagaaa gttg             294
<210> 6
   <211> 330
   <212> DNA
   <213> Homo sapiens
<400> 6
   cacctgaact cctgggggga ccgtcagtct tcctcttccc cccaaaaccc aaggacaccc        60
   tcatgatctc ccggacccct gaggtcacat gcgtggtggt ggacgtgagc cacgaagacc       120
   ctgaggtcaa gttcaactgg tacgtggacg gcgtggaggt gcataatgcc aagacaaagc       180
   cgcgggagga gcagtacaac agcacgtacc gtgtggtcag cgtcctcacc gtcctgcacc       240
   aggactggct gaatggcaag gagtacaagt gcaaggtctc caacaaagcc ctcccagccc       300
   ccatcgagaa aaccatctcc aaagccaaag                                        330
<210> 7
   <211> 320
   <212> DNA
   <213> Homo sapiens
<400> 7
   ggcagccccg agaaccacag gtgtacaccc tgcccccatc ccgggatgag ctgaccaaga        60
   accaggtcag cctgacctgc ctggtcaaag gcttctatcc cagcgacatc gccgtggagt       120
   gggagagcaa tgggcagccg gagaacaact acaagaccac gcctcccgtg ctggactccg       180
   acggctcctt cttcctctac agcaagctca ccgtggacaa gagcaggtgg cagcagggga       240
   acgtcttctc atgctccgtg atgcatgagg ctctgcacaa ccactacacg cagaagagcc       300
   tctccctgtc tccgggtaaa                                                   320
<210> 8
   <211> 8778
   <212> DNA
   <213> Artificial Sequence
<220>
   <223> plasmid
<400> 8
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattaattg catgaagaat 180
   ctgcttaggg ttaggcgttt tgcgctgctt cgcgatgtac gggccagata tacgcgttga 240
   cattgattat tgactagtta ttaatagtaa tcaattacgg ggtcattagt tcatagccca 300
   tatatggagt tccgcgttac ataacttacg gtaaatggcc cgcctggctg accgcccaac 360
   gacccccgcc cattgacgtc aataatgacg tatgttccca tagtaacgcc aatagggact 420
   ttccattgac gtcaatgggt ggactattta cggtaaactg cccacttggc agtacatcaa 480
   gtgtatcata tgccaagtac gccccctatt gacgtcaatg acggtaaatg gcccgcctgg 540
   cattatgccc agtacatgac cttatgggac tttcctactt ggcagtacat ctacgtatta 600
   gtcatcgcta ttaccatggt gatgcggttt tggcagtaca tcaatgggcg tggatagcgg 660
   tttgactcac ggggatttcc aagtctccac cccattgacg tcaatgggag tttgttttgg 720
   caccaaaatc aacgggactt tccaaaatgt cgtaacaact ccgccccatt gacgcaaatg 780
   ggcggtaggc gtgtacggtg ggaggtctat ataagcagag ctctctggct aactagagaa 840
   cccactgctt actggcttat cgaaattaat acgactcact atagggagac ccaagctagc 900
   ttggtaccgg gacctcacca tgggatggag ctgtatcatc ctcttcttgg tagcaacagc 960
   tacaggtaag gggctcacag tagcaggctt gaggtctgga catatatatg ggtgacaatg 1020
   acatccactt tgcctttctc tccacaggtg tgcattccca ggtccaattg cagcagcctg 1080
   gggctgagct tgtgaagcct ggggcttcag tgaagctgtc ctgcaaggct tctggctaca 1140
   ccttcaccag ctactggatg cactgggtga agcagaggcc tggacgaggc cttgagtgga 1200
   ttggaaggat tgatcctaat agtggtggta ctaagtacaa tgagaagttc aagagcaagg 1260
   ccacactgac tgtagacaaa ccctccagca cagcctacat gcagctcagc agcctgacat 1320
   ctgaggactc tgcggtctat tattgtgcaa gatacgatta ctacggtagt agctactttg 1380
   actactgggg ccaaggcacc actctcacag tctcctcagg tgagttaacg tacgctagca 1440
   agctttctgg ggcaggccag gcctgacctt ggctttgggg cagggagggg gctaaggtga 1500
   ggcaggtggc gccagccagg tgcacaccca atgcccatga gcccagacac tggacgctga 1560
   acctcgcgga cagttaagaa cccaggggcc tctgcgccct gggcccagct ctgtcccaca 1620
   ccgcggtcac atggcaccac ctctcttgca gcctccacca agggcccatc ggtcttcccc 1680
   ctggcaccct cctccaagag cacctctggg ggcacagcgg ccctgggctg cctggtcaag 1740
   gactacttcc ccgaaccggt gacggtgtcg tggaactcag gcgccctgac cagcggcgtg 1800
   cacaccttcc cggctgtcct acagtcctca ggactctact ccctcagcag cgtggtgacc 1860
   gtgccctcca gcagcttggg cacccagacc tacatctgca acgtgaatca caagcccagc 1920
   aacaccaagg tggacaagaa agttggtgag aggccagcac agggagggag ggtgtctgct 1980
   ggaagccagg ctcagcgctc ctgcctggac gcatcccggc tatgcagccc cagtccaggg 2040
   cagcaaggca ggccccgtct gcctcttcac ccggaggcct ctgcccgccc cactcatgct 2100
   cagggagagg gtcttctggc tttttcccca ggctctgggc aggcacaggc taggtgcccc 2160
   taacccaggc cctgcacaca aaggggcagg tgctgggctc agacctgcca agagccatat 2220
   ccgggaggac cctgcccctg acctaagccc accccaaagg ccaaactctc cactccctca 2280
   gctcggacac cttctctcct cccagattcc agtaactccc aatcttctct ctgcagagcc 2340
   caaatcttgt gacaaaactc acacatgccc accgtgccca ggtaagccag cccaggcctc 2400
   gccctccagc tcaaggcggg acaggtgccc tagagtagcc tgcatccagg gacaggcccc 2460
   agccgggtgc tgacacgtcc acctccatct cttcctcagc acctgaactc ctggggggac 2520
   cgtcagtctt cctcttcccc ccaaaaccca aggacaccct catgatctcc cggacccctg 2580
   aggtcacatg cgtggtggtg gacgtgagcc acgaagaccc tgaggtcaag ttcaactggt 2640
   acgtggacgg cgtggaggtg cataatgcca agacaaagcc gcgggaggag cagtacaaca 2700
   gcacgtaccg tgtggtcagc gtcctcaccg tcctgcacca ggactggctg aatggcaagg 2760
   agtacaagtg caaggtctcc aacaaagccc tcccagcccc catcgagaaa accatctcca 2820
   aagccaaagg tgggacccgt ggggtgcgag ggccacatgg acagaggccg gctcggccca 2880
   ccctctgccc tgagagtgac cgctgtacca acctctgtcc ctacagggca gccccgagaa 2940
   ccacaggtgt acaccctgcc cccatcccgg gatgagctga ccaagaacca ggtcagcctg 3000
   acctgcctgg tcaaaggctt ctatcccagc gacatcgccg tggagtggga gagcaatggg 3060
   cagccggaga acaactacaa gaccacgcct cccgtgctgg actccgacgg ctccttcttc 3120
   ctctacagca agctcaccgt ggacaagagc aggtggcagc aggggaacgt cttctcatgc 3180
   tccgtgatgc atgaggctct gcacaaccac tacacgcaga agagcctctc cctgtctccg 3240
   ggtaaatgag tgcgacggcc ggcaagcccc cgctccccgg gctctcgcgg tcgcacgagg 3300
   atgcttggca cgtaccccct gtacatactt cccgggcgcc cagcatggaa ataaagcacc 3360
   cagcgctgcc ctgggcccct gcgagactgt gatggttctt tccacgggtc aggccgagtc 3420
   tgaggcctga gtggcatgag ggaggcagag cgggtcccac tgtccccaca ctggcccagg 3480
   ctgtgcaggt gtgcctgggc cccctagggt ggggctcagc caggggctgc cctcggcagg 3540
   gtgggggatt tgccagcgtg gccctccctc cagcagcacc tgccctgggc tgggccacgg 3600
   gaagccctag gagcccctgg ggacagacac acagcccctg cctctgtagg agactgtcct 3660
   gttctgtgag cgcccctgtc ctcccgacct ccatgcccac tcgggggcat gcctagtcca 3720
   tgtgcgtagg gacaggccct ccctcaccca tctaccccca cggcactaac ccctggctgc 3780
   cctgcccagc ctcgcacccg catggggaca caaccgactc cggggacatg cactctcggg 3840
   ccctgtggag ggactggtgc agatgcccac acacacactc agcccagacc cgttcaacaa 3900
   accccgcact gaggttggcc ggccacacgg ccaccacaca cacacgtgca cgcctcacac 3960
   acggagcctc acccgggcga actgcacagc acccagacca gagcaaggtc ctcgcacacg 4020
   tgaacactcc tcggacacag gcccccacga gccccacgcg gcacctcaag gcccacgagc 4080
   ctctcggcag cttctccaca tgctgacctg ctcagacaaa cccagccctc ctctcacaag 4140
   ggtgcccctg cagccgccac acacacacag gggatcacac accacgtcac gtccctggcc 4200
   ctggcccact tcccagtgcc gcccttccct gcagacggat ccactagtaa cggccgccag 4260
   tgtgctggaa ttctgcagat atccatcaca ctggcggccg ctcgagcatg catctagagg 4320
   gccctattct atagtgtcac ctaaatgcta gagctcgctg atcagcctcg actgtgcctt 4380
   ctagttgcca gccatctgtt gtttgcccct cccccgtgcc ttccttgacc ctggaaggtg 4440
   ccactcccac tgtcctttcc taataaaatg aggaaattgc atcgcattgt ctgagtaggt 4500
   gtcattctat tctggggggt ggggtggggc aggacagcaa gggggaggat tgggaagaca 4560
   atagcaggca tgctggggat gcggtgggct ctatggcttc tgaggcggaa agaaccagct 4620
   ggggctctag ggggtatccc cacgcgccct gtagcggcgc attaagcgcg gcgggtgtgg 4680
   tggttacgcg cagcgtgacc gctacacttg ccagcgccct agcgcccgct cctttcgctt 4740
   tcttcccttc ctttctcgcc acgttcgccg gctttccccg tcaagctcta aatcggggca 4800
   tccctttagg gttccgattt agtgctttac ggcacctcga ccccaaaaaa cttgattagg 4860
   gtgatggttc acgtagtggg ccatcgccct gatagacggt ttttcgccct ttgacgttgg 4920
   agtccacgtt ctttaatagt ggactcttgt tccaaactgg aacaacactc aaccctatct 4980
   cggtctattc ttttgattta taagggattt tggggatttc ggcctattgg ttaaaaaatg 5040
   agctgattta acaaaaattt aacgcgaatt aattctgtgg aatgtgtgtc agttagggtg 5100
   tggaaagtcc ccaggctccc caggcaggca gaagtatgca aagcatgcat ctcaattagt 5160
   cagcaaccag gtgtggaaag tccccaggct ccccagcagg cagaagtatg caaagcatgc 5220
   atctcaatta gtcagcaacc atagtcccgc ccctaactcc gcccatcccg cccctaactc 5280
   cgcccagttc cgcccattct ccgccccatg gctgactaat tttttttatt tatgcagagg 5340
   ccgaggccgc ctctgcctct gagctattcc agaagtagtg aggaggcttt tttggaggcc 5400
   taggcttttg caaaaagctc ccgggagctt gtatatccat tttcggatct gatcaagaga 5460
   caggatgagg atcgtttcgc atgattgaac aagatggatt gcacgcaggt tctccggccg 5520
   cttgggtgga gaggctattc ggctatgact gggcacaaca gacaatcggc tgctctgatg 5580
   ccgccgtgtt ccggctgtca gcgcaggggc gcccggttct ttttgtcaag accgacctgt 5640
   ccggtgccct gaatgaactg caggacgagg cagcgcggct atcgtggctg gccacgacgg 5700
   gcgttccttg cgcagctgtg ctcgacgttg tcactgaagc gggaagggac tggctgctat 5760
   tgggcgaagt gccggggcag gatctcctgt catctcacct tgctcctgcc gagaaagtat 5820
   ccatcatggc tgatgcaatg cggcggctgc atacgcttga tccggctacc tgcccattcg 5880
   accaccaagc gaaacatcgc atcgagcgag cacgtactcg gatggaagcc ggtcttgtcg 5940
   atcaggatga tctggacgaa gagcatcagg ggctcgcgcc agccgaactg ttcgccaggc 6000
   tcaaggcgcg catgcccgac ggcgaggatc tcgtcgtgac ccatggcgat gcctgcttgc 6060
   cgaatatcat ggtggaaaat ggccgctttt ctggattcat cgactgtggc cggctgggtg 6120
   tggcggaccg ctatcaggac atagcgttgg ctacccgtga tattgctgaa gagcttggcg 6180
   gcgaatgggc tgaccgcttc ctcgtgcttt acggtatcgc cgctcccgat tcgcagcgca 6240
   tcgccttcta tcgccttctt gacgagttct tctgagcggg actctggggt tcgaaatgac 6300
   cgaccaagcg acgcccaacc tgccatcacg agatttcgat tccaccgccg ccttctatga 6360
   aaggttgggc ttcggaatcg ttttccggga cgccggctgg atgatcctcc agcgcgggga 6420
   tctcatgctg gagttcttcg cccaccccaa cttgtttatt gcagcttata atggttacaa 6480
   ataaagcaat agcatcacaa atttcacaaa taaagcattt ttttcactgc aaattctagt 6540
   tgtggtttgt ccaaactcat caatgtatct tatcatgtct gtataccgtc gacctctagc 6600
   tagagcttgg cgtaatcatg gtcatagctg tttcctgtgt gaaattgtta tccgctcaca 6660
   attccacaca acatacgagc cggaagcata aagtgtaaag cctggggtgc ctaatgagtg 6720
   agctaactca cattaattgc gttgcgctca ctgcccgctt tccagtcggg aaacctgtcg 6780
   tgccagctgc attaatgaat cggccaacgc gcggggagag gcggtttgcg tattgggcgc 6840
   tcttccgctt cctcgctcac tgactcgctg cgctcggtcg ttcggctgcg gcgagcggta 6900
   tcagctcact caaaggcggt aatacggtta tccacagaat caggggataa cgcaggaaag 6960
   aacatgtgag caaaaggcca gcaaaaggcc aggaaccgta aaaaggccgc gttgctggcg 7020
   tttttccata ggctccgccc ccctgacgag catcacaaaa atcgacgctc aagtcagagg 7080
   tggcgaaacc cgacaggact ataaagatac caggcgtttc cccctggaag ctccctcgtg 7140
   cgctctcctg ttccgaccct gccgcttacc ggatacctgt ccgcctttct cccttcggga 7200
   agcgtggcgc tttctcaatg ctcacgctgt aggtatctca gttcggtgta ggtcgttcgc 7260
   tccaagctgg gctgtgtgca cgaacccccc gttcagcccg accgctgcgc cttatccggt 7320
   aactatcgtc ttgagtccaa cccggtaaga cacgacttat cgccactggc agcagccact 7380
   ggtaacagga ttagcagagc gaggtatgta ggcggtgcta cagagttctt gaagtggtgg 7440
   cctaactacg gctacactag aaggacagta tttggtatct gcgctctgct gaagccagtt 7500
   accttcggaa aaagagttgg tagctcttga tccggcaaac aaaccaccgc tggtagcggt 7560
   ggtttttttg tttgcaagca gcagattacg cgcagaaaaa aaggatctca agaagatcct 7620
   ttgatctttt ctacggggtc tgacgctcag tggaacgaaa actcacgtta agggattttg 7680
   gtcatgagat tatcaaaaag gatcttcacc tagatccttt taaattaaaa atgaagtttt 7740
   aaatcaatct aaagtatata tgagtaaact tggtctgaca gttaccaatg cttaatcagt 7800
   gaggcaccta tctcagcgat ctgtctattt cgttcatcca tagttgcctg actccccgtc 7860
   gtgtagataa ctacgatacg ggagggctta ccatctggcc ccagtgctgc aatgataccg 7920
   cgagacccac gctcaccggc tccagattta tcagcaataa accagccagc cggaagggcc 7980
   gagcgcagaa gtggtcctgc aactttatcc gcctccatcc agtctattaa ttgttgccgg 8040
   gaagctagag taagtagttc gccagttaat agtttgcgca acgttgttgc cattgctaca 8100
   ggcatcgtgg tgtcacgctc gtcgtttggt atggcttcat tcagctccgg ttcccaacga 8160
   tcaaggcgag ttacatgatc ccccatgttg tgcaaaaaag cggttagctc cttcggtcct 8220
   ccgatcgttg tcagaagtaa gttggccgca gtgttatcac tcatggttat ggcagcactg 8280
   cataattctc ttactgtcat gccatccgta agatgctttt ctgtgactgg tgagtactca 8340
   accaagtcat tctgagaata gtgtatgcgg cgaccgagtt gctcttgccc ggcgtcaata 8400
   cgggataata ccgcgccaca tagcagaact ttaaaagtgc tcatcattgg aaaacgttct 8460
   tcggggcgaa aactctcaag gatcttaccg ctgttgagat ccagttcgat gtaacccact 8520
   cgtgcaccca actgatcttc agcatctttt actttcacca gcgtttctgg gtgagcaaaa 8580
   acaggaaggc aaaatgccgc aaaaaaggga ataagggcga cacggaaatg ttgaatactc 8640
   atactcttcc tttttcaata ttattgaagc atttatcagg gttattgtct catgagcgga 8700
   tacatatttg aatgtattta gaaaaataaa caaatagggg ttccgcgcac atttccccga 8760
   aaagtgccac ctgacgtc 8778
<210> 9
   <211> 319
   <212> DNA
   <213> Homo sapiens
<400> 9
   aactgtggct gcaccatctg tcttcatctt cccgccatct gatgagcagt tgaaatctgg       60
   aactgcctct gttgtgtgcc tgctgaataa cttctatccc agagaggcca aagtacagtg      120
   gaaggtggat aacgccctcc aatcgggtaa ctcccaggag agtgtcacag agcaggacag      180
   caaggacagc acctacagcc tcagcagcac cctgacgctg agcaaagcag actacgagaa      240
   acacaaagtc tacgcctgcg aagtcaccca tcagggcctg agctcgcccg tcacaaagag      300
   cttcaacagg ggagagtgt                                                   319
<210> 10
   <211> 1479
   <212> DNA
   <213> Artificial Sequence
<220>
   <223> chimeric
<400> 10
   gaattcgccg ccaccatggg atggagctgt atcatcctct tcttggtagc aacagctaca 60
   ggtgtccact ccgagatcca gctgcagcag tctggacctg agctggtgaa gcctggggct 120
   tcagtgaagg tatcctgcaa ggcttctggt tactcattca ctgactacaa catgtactgg 180
   gtgaagcaga gccatggaaa gagccttgag tggattggat atattgatcc ttacaatggt 240
   gatactacct acaaccagaa gttcaagggc aaggccacat tgactgttga caagtcctcc 300
   agcacagcct tcatccatct caacagcctg acatctgagg actctgcagt ctattactgt 360
   gcaagatccc cttatggtca ctatgctatg gactactggg gtcaaggaac ctcagtcacc 420
   gtctcctcag ctagcaccaa gggcccatcg gtcttccccc tggcgccctg ctccaggagc 480
   acccctgggg gcacagcggc cctgggctgc ctggtcaagg actacttccc cgaaccggtg 540
   acggtgtcgt ggaactcagg cgccctgacc agcggcgtgc acaccttccc ggctgtccta 600
   cagtcctcag gactctactc cctcagcagc gtggtgaccg tgccctccag cagcttgggc 660
   acccagacct acacctgcaa cgtgaatcac aagcccagca acaccaaggt ggacaagaga 720
   gttgagctca aaaccccact tggtgacaca actcacacat gcccacggtg cccagagccc 780
   aaatcttgtg acacacctcc cccatgccca cggtgcccag cacctgaact cctgggagga 840
   ccgtcagtct tcctcttccc cccaaaaccc aaggataccc ttatgatttc ccggacccct 900
   gaggtcacgt gcgtggtggt ggacgtgagc cacgaagacc ccgaggtcca gttcaagtgg 960
   tacgtggacg gcgtggaggt gcataatgcc aagacaaagc cgcgggagga gcagttcaac 1020
   agcacgttcc gtgtggtcag cgtcctcacc gtcctgcacc aggactggct gaacggcaag 1080
   gagtacaagt gcaaggtctc caacaaagcc ctcccagccc ccatcgagaa aaccatctcc 1140
   aaaaccaaag gacagccccg agaaccacag gtgtacaccc tgcccccatc ccgggaggag 1200
   atgaccaaga accaggtcag cctgacctgc ctggtcaaag gcttctaccc cagcgacatc 1260
   gccgtggagt gggagagcag cgggcagccg gagaacaact acaacaccac gcctcccatg 1320
   ctggactccg acggctcctt cttcctctac agcaagctca ccgtggacaa gagcaggtgg 1380
   cagcagggga acgtcttctc atgctccgtg atgcatgagg ctctgcacaa ccactacacg 1440
   cagaagagcc tctccctgtc tccgggtaaa tgaaagctt 1479
<210> 11
   <211> 485
   <212> PRT
   <213> Artificial Sequence
<220>
   <223> Chimeric
<400> 11
   Met Gly Trp Ser Cys Ile Ile Leu Phe Leu Val Ala Thr Ala Thr Gly
   1               5                   10                  15
   Val His Ser Glu Ile Gln Leu Gln Gln Ser Gly Pro Glu Leu Val Lys
               20                  25                  30
   Pro Gly Ala Ser Val Lys Val Ser Cys Lys Ala Ser Gly Tyr Ser Phe
           35                  40                  45
   Thr Asp Tyr Asn Met Tyr Trp Val Lys Gln Ser His Gly Lys Ser Leu
       50                  55                  60
   Glu Trp Ile Gly Tyr Ile Asp Pro Tyr Asn Gly Asp Thr Thr Tyr Asn
   65                  70                  75                  80
   Gln Lys Phe Lys Gly Lys Ala Thr Leu Thr Val Asp Lys Ser Ser Ser
                   85                  90                  95
   Thr Ala Phe Ile His Leu Asn Ser Leu Thr Ser Glu Asp Ser Ala Val
               100                 105                 110
   Tyr Tyr Cys Ala Arg Ser Pro Tyr Gly His Tyr Ala Met Asp Tyr Trp
           115                 120                 125
   Gly Gln Gly Thr Ser Val Thr Val Ser Ser Ala Ser Thr Lys Gly Pro
       130                 135                 140
   Ser Val Phe Pro Leu Ala Pro Cys Ser Arg Ser Thr Pro Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Thr Cys Asn Val Asn
       210                 215                 220
   His Lys Pro Ser Asn Thr Lys Val Asp Lys Arg Val Glu Leu Lys Thr
   225                 230                 235                 240
   Pro Leu Gly Asp Thr Thr His Thr Cys Pro Arg Cys Pro Glu Pro Lys
                   245                 250                 255
   Ser Cys Asp Thr Pro Pro Pro Cys Pro Arg Cys Pro Ala Pro Glu Leu
               260                 265                 270
   Leu Gly Gly Pro Ser Val Phe Leu Phe Pro Pro Lys Pro Lys Asp Thr
           275                 280                 285
   Leu Met Ile Ser Arg Thr Pro Glu Val Thr Cys Val Val Val Asp Val
       290                 295                 300
   Ser His Glu Asp Pro Glu Val Gln Phe Lys Trp Tyr Val Asp Gly Val
   305                 310                 315                 320
   Glu Val His Asn Ala Lys Thr Lys Pro Arg Glu Glu Gln Phe Asn Ser
                   325                 330                 335
   Thr Phe Arg Val Val Ser Val Leu Thr Val Leu His Gln Asp Trp Leu
               340                 345                 350
   Asn Gly Lys Glu Tyr Lys Cys Lys Val Ser Asn Lys Ala Leu Pro Ala
           355                 360                 365
   Pro Ile Glu Lys Thr Ile Ser Lys Thr Lys Gly Gln Pro Arg Glu Pro
       370                 375                 380
   Gln Val Tyr Thr Leu Pro Pro Ser Arg Glu Glu Met Thr Lys Asn Gln
   385                 390                 395                 400
   Val Ser Leu Thr Cys Leu Val Lys Gly Phe Tyr Pro Ser Asp Ile Ala
                   405                 410                 415
   Val Glu Trp Glu Ser Ser Gly Gln Pro Glu Asn Asn Tyr Asn Thr Thr
               420                 425                 430
   Pro Pro Met Leu Asp Ser Asp Gly Ser Phe Phe Leu Tyr Ser Lys Leu
           435                 440                 445
   Thr Val Asp Lys Ser Arg Trp Gln Gln Gly Asn Val Phe Ser Cys Ser
       450                 455                 460
   Val Met His Glu Ala Leu His Asn His Tyr Thr Gln Lys Ser Leu Ser
   465 470 475 480
   Leu Ser Pro Gly Lys
   485
<210> 12
   <211> 723
   <212> DNA
   <213> Artificial Sequence
<220>
   <223> Chimeric
<400> 12
   gaattcgccg ccaccatggg atggagctgt atcatcctct ttttggttgc aacagctaca      60
   ggtgtccact ccgacattgt gatgacccag tctcacaaac tcttgtccac atcagtagga     120
   gacagggtca gcatcacctg caaggccagt caggatgtga gtactgctgt agactggtat     180
   caacagaaac caggacaatc tcctaaacta ctgattaact gggcatccac ccggcacact     240
   ggagtccctg atcgcttcac aggcagtgga tctgggacag attatactct caccatcagc     300
   agtatgcagg ctgaagacct ggcactttat tactgtcgac aacattatag cactccattc     360
   acgttcggct cggggacaaa gttggaaata aaacgaactg tggctgcacc atctgtcttc     420
   atcttcccgc catctgatga gcagttgaaa tctggaactg cctctgttgt gtgcctgctg     480
   aataacttct atcccagaga ggccaaagta cagtggaagg tggataacgc cctccaatcg     540
   ggtaactccc aggagagtgt cacagagcag gacagcaagg acagcaccta cagcctcagc     600
   agcaccctga cgctgagcaa agcagactac gagaaacaca aagtctacgc ctgcgaagtc     660
   acccatcagg gcctgagctc gcccgtcaca aagagcttca acaggggaga gtgttgaaag     720
   ctt                                                                   723
<210> 13
   <211> 233
   <212> PRT
   <213> Artificial Sequence
<220>
   <223> Chimeric
<400> 13
   Met Gly Trp Ser Cys Ile Ile Leu Phe Leu Val Ala Thr Ala Thr Gly
   1               5                   10                  15
   Val His Ser Asp Ile Val Met Thr Gln Ser His Lys Leu Leu Ser Thr
               20                  25                  30
   Ser Val Gly Asp Arg Val Ser Ile Thr Cys Lys Ala Ser Gln Asp Val
           35                  40                  45
   Ser Thr Ala Val Asp Trp Tyr Gln Gln Lys Pro Gly Gln Ser Pro Lys
       50                  55                  60
   Leu Leu Ile Asn Trp Ala Ser Thr Arg His Thr Gly Val Pro Asp Arg
   65                  70                  75                  80
   Phe Thr Gly Ser Gly Ser Gly Thr Asp Tyr Thr Leu Thr Ile Ser Ser
                   85                  90                  95
   Met Gln Ala Glu Asp Leu Ala Leu Tyr Tyr Cys Arg Gln His Tyr Ser
               100                 105                 110
   Thr Pro Phe Thr Phe Gly Ser Gly Thr Lys Leu Glu Ile Lys Arg Thr
           115                 120                 125
   Val Ala Ala Pro Ser Val Phe Ile Phe Pro Pro Ser Asp Glu Gln Leu
       130                 135                 140
   Lys Ser Gly Thr Ala Ser Val Val Cys Leu Leu Asn Asn Phe Tyr Pro
   145                 150                 155                 160
   Arg Glu Ala Lys Val Gln Trp Lys Val Asp Asn Ala Leu Gln Ser Gly
                   165                 170                 175
   Asn Ser Gln Glu Ser Val Thr Glu Gln Asp Ser Lys Asp Ser Thr Tyr
               180                 185                 190
   Ser Leu Ser Ser Thr Leu Thr Leu Ser Lys Ala Asp Tyr Glu Lys His
           195                 200                 205
   Lys Val Tyr Ala Cys Glu Val Thr His Gln Gly Leu Ser Ser Pro Val
       210                 215                 220
   Thr Lys Ser Phe Asn Arg Gly Glu Cys
   225                 230
<210> 14
   <211> 377
   <212> PRT
   <213> Artificial Sequence
<220>
   <223> Chimeric
<400> 14
   Ala Ser Thr Lys Gly Pro Ser Val Phe Pro Leu Ala Pro Cys Ser Arg
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Thr Cys Asn Val Asn His Lys Pro Ser Asn Thr Lys Val Asp Lys
                   85                  90                  95
   Arg Val Glu Leu Lys Thr Pro Leu Gly Asp Thr Thr His Thr Cys Pro
               100                 105                 110
   Arg Cys Pro Glu Pro Lys Ser Cys Asp Thr Pro Pro Pro Cys Pro Arg
           115                 120                 125
   Cys Pro Glu Pro Lys Ser Cys Asp Thr Pro Pro Pro Cys Pro Arg Cys
       130                 135                 140
   Pro Glu Pro Lys Ser Cys Asp Thr Pro Pro Pro Cys Pro Arg Cys Pro
   145                 150                 155                 160
   Ala Pro Glu Leu Leu Gly Gly Pro Ser Val Phe Leu Phe Pro Pro Lys
                   165                 170                 175
   Pro Lys Asp Thr Leu Met Ile Ser Arg Thr Pro Glu Val Thr Cys Val
               180                 185                 190
   Val Val Asp Val Ser His Glu Asp Pro Glu Val Gln Phe Lys Trp Tyr
           195                 200                 205
   Val Asp Gly Val Glu Val His Asn Ala Lys Thr Lys Pro Arg Glu Glu
       210                 215                 220
   Gln Tyr Asn Ser Thr Phe Arg Val Val Ser Val Leu Thr Val Leu His
   225                 230                 235                 240
   Gln Asp Trp Leu Asn Gly Lys Glu Tyr Lys Cys Lys Val Ser Asn Lys
                   245                 250                 255
   Ala Leu Pro Ala Pro Ile Glu Lys Thr Ile Ser Lys Thr Lys Gly Gln
                       260                 265                 270
   Pro Arg Glu Pro Gln Val Tyr Thr Leu Pro Pro Ser Arg Glu Glu Met
           275                 280                 285
   Thr Lys Asn Gln Val Ser Leu Thr Cys Leu Val Lys Gly Phe Tyr Pro
       290                 295                 300
   Ser Asp Ile Ala Val Glu Trp Glu Ser Ser Gly Gln Pro Glu Asn Asn
   305                 310                 315                 320
   Tyr Asn Thr Thr Pro Pro Met Leu Asp Ser Asp Gly Ser Phe Phe Leu
                   325                 330                 335
   Tyr Ser Lys Leu Thr Val Asp Lys Ser Arg Trp Gln Gln Gly Asn Ile
               340                 345                 350
   Phe Ser Cys Ser Val Met His Glu Ala Leu His Asn Arg Phe Thr Gln
           355                 360                 365
   Lys Ser Leu Ser Leu Ser Pro Gly Lys
       370                 375